# EUROPÄISCHE PATENTSCHRIFT

(11) **EP 0 740 666 B1**
(45) Veröffentlichungstag und Bekanntmachung des Hinweises auf die Patenterteilung: **10.11.1999**
(21) Anmeldenummer: 95905616.9
(22) Anmeldetag: 09.01.1995
(51) Int. Cl.: C07D 401/12, A01N 43/54, C07D 417/12, C07D 487/04

(54) **SUBSTITUIERTE 1,2,3,4-TETRAHYDRO-5-NITRO-PYRIMIDINE**
SUBSTITUTED 1,2,3,4-TETRAHYDRO-5-NITRO PYRIMIDINES
1,2,3,4-TETRAHYDRO-5-NITRO-PYRIMIDINES SUBSTITUEES

(30) Priorität: 21.01.1994 DE 4401635
(43) Veröffentlichungstag der Anmeldung: 06.11.1996
(73) Patentinhaber: BAYER AG, 51368 Leverkusen (DE)
(72) Erfinder: KRÜGER, Bernd-Wieland, D-51467 Bergisch Gladbach (DE); UHR, Hermann, D-47800 Krefeld (DE); KANELLAKOPULOS, Johannes, D-40724 Hilden (DE); GESING, Ernst, Rudolf, D-40699 Erkrath-Hochdahl (DE); WOLF, Hilmar, D-40764 Langenfeld (DE); TURBERG, Andreas, D-40699 Erkrath (DE); MENCKE, Norbert, D-51381 Leverkusen (DE); ERDELEN, Christoph, D-42799 Leichlingen (DE); WACHENDORFF-NEUMANN, Ulrike, D-40789 Monheim (DE); HARTWIG, Jürgen, D-42799 Leichlingen (DE)
(86) Internationale Anmeldenummer: EP9500058
(87) Internationale Veröffentlichungsnummer: WO9519977

(56) Entgegenhaltungen:
- EP-A- 0 247 477
- EP-A- 0 316 843
- EP-A- 0 316 845
- EP-A- 0 366 085
- EP-A- 0 375 613
- EP-A- 0 398 084
- WO-A-90/05134
- WO-A-94/05670
- JP-A- 3 264 580

## Beschreibung

Die vorliegende Erfindung betrifft neue substituierte 1,2,3,4-Tetrahydro-5-nitro-pyrimidine, Verfahren zu ihrer Herstellung und ihre Verwendung in Schädlingsbekämpfungsmitteln, insbesondere als Insektizide.

Darüber hinaus besitzen die neuen Verbindungen eine stark ausgeprägte ektoparasitizide Wirksamkeit.

Weiterhin ist bekannt, daß bestimmte nitrierte Stickstoffheterocyclen insektizide Eigenschaften besitzen (vgl. EP-0 316 843; EP-0 375 613; WO-A-94/05 670; WO-A-90/05 134; EP-A-0 398 084; EP-A-366 085; EP-A-0 247 477; JP-A-3 264 580). Die EP-0 316 843, EP-0 366 085, WO-A-94/05 670, WO-A-90/05 134, EP-A-0 398 084, EP-A-0 366 085, EP-A-0 247 477 sowie die JP-A-3 264 580 beschreiben insbesondere verschiedenartig substituierte Nitro-tetrahydro-pyrimidine.

Es wurden die neuen substituierten 1,2,3,4-Tetrahydro-5-nitro-pyrimidine der Formel (I) gefunden. 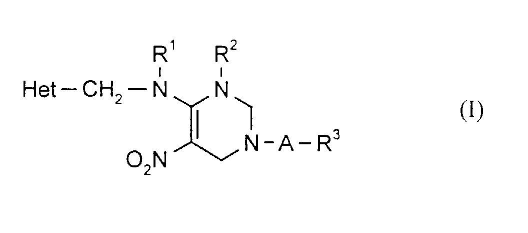 in welcher
- Het: für gegebenenfalls durch Halogen substituiertes Pyridyl oder für gegebenenfalls durch Halogen substituiertes Thiazolyl steht und
- R¹: für Methyl oder Ethyl steht,
- R²: für Methyl oder Ethyl steht,
- R¹ und R²: gemeinsam mit den angrenzenden Atomen einen gesättigten 5- oder 6-Ring bilden der gegebenenfalls durch OH oder C₁₋₄-Alkyl substituiert ist, wie z.B. 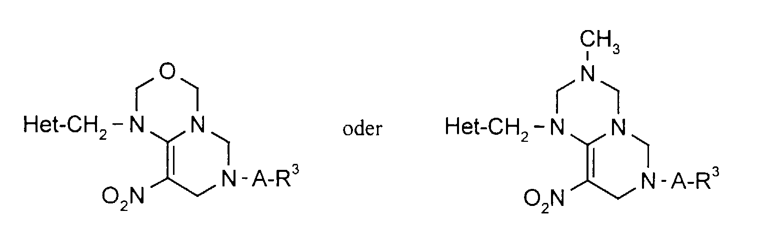 wobei
Het, A und R³ die angegebenen Bedeutungen haben,
- A: für geradkettiges oder verzweigtes Alkylen mit 2 bis 6 C-Atomen steht, das gegebenenfalls durch Phenyl, Halogen, OH, CN, Amino, Mono- oder Di-C₁₋₄-alkylamino substituiert sein kann, weiter steht A für Cyclopropylen, Methylcyclopropylen, Cyclohexylen, Cyclooctylen,
- R³: für einen der Reste 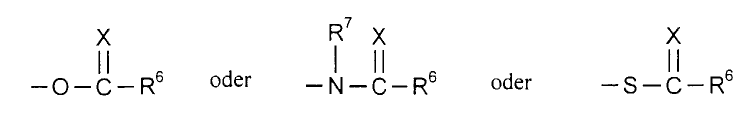 steht,
wobei
- R⁶: für C₁₋₈-Alkyl, C₃₋₈-Cycloalkyl, C₂₋₈-Alkenyl, Phenyl, Benzyl, Pyridyl, Imidazolyl, Pyrazolyl, Thiazolyl, Oxazolyl, Pyrrolyl, Phenylethyl, C₁₋₈-Alkoxy, C₃₋₈-Cycloalkoxy, C₃₋₈-Alkenoxy, Phenoxy, Benzyloxy, Phenylethyloxy, C₁₋₈-Alkylthio, Phenylthio, Benzylthio, Phenylethylthio, Amino, C₁₋₄-Alkylamino, Di-C₁₋₄-alkylamino, Phenylamino, N-Phenyl-N-C₁₋₄-alkylamino, Phenyl-C₁₋₄-alkyl-amino, N-Phenyl-C₁₋₄-alkyl-N-C₁₋₄-alkyl-amino steht, wobei diese Reste gegebenenfalls durch einen oder mehrere gleiche oder verschiedene Reste aus der Reihe Alkyl mit 1 bis 4 Kohlenstoffatomen, wie Methyl, Ethyl, n- und i-Propyl und n-, i- und t-Butyl; Cycloalkyl, Cycloalkylamino oder Cycloalkylalkyl mit 3 bis 7 Kohlenstoffatomen im Cycloalkylteil und 1 bis 5 Kohlenstoffatome im geradkettigen oder verzweigten Alkylteil; Phenylalkyl mit 1 bis 4 Kohlenstoffatomen im Alkylteil; Alkoxy mit 1 bis 4 Kohlenstoffatomen, wie Methoxy, Ethoxy, n- und i-Propoxy und n-, i- und t-Butoxy, Alkylthio mit 1 bis 4 Kohlenstoffatomen, wie Methylthio, Ethylthio, n- und i-Propylthio und n-, i- und t-Butylthio; Halogenalkyl mit 1 bis 4 Kohlenstoffatomen und 1 bis 5 Halogenatomen, wobei die Halogenatome gleich oder verschieden sind und als Halogenatome Fluor, Chlor oder Brom stehen. Hydroxy; Halogen, Fluor, Chlor, Brom und Jod. Cyano; Nitro; Amino; Monoalkyl- und Dialkylamino oder Mono- und Dialkylaminocarbonyl mit 1 bis 4 Kohlenstoffatomen je Alkylgruppe, wie Methylamino, Carboxy; Carbalkoxy mit 2 bis 4 Kohlenstoffatomen, wie Carbomethoxy und Carboethoxy; Sulfo (-SO₃H), Alkylsulfonyl mit 1 bis 4 Kohlenstoffatomen. wie Methylsulfonyl und Ethylsulfonyl; Arylsulfonyl mit 6 oder 10 Arylkohlenstoffatomen, wie Phenylsulfonyl substituiert sein können.
- R⁷: für Wasserstoff oder C₁-C₄-Alkyl steht und
- X: für Sauerstoff oder Schwefel steht,
ausgenommen die Verbindungen der Formel (I), in welcher Het für 6-chlor-pyrid-3-yl, A für (CH₂)₂, R³ für N(R⁷)-C(X)-R⁶, R⁷ für H, R⁶ für Methyl, X für Sauerstoff und R¹ und R² gemeinsam für (CH₂)₂ stehen.

Weiterhin wurde gefunden, daß man die substituierten 1,2,3,4-Tetrahydro-5-nitropyrimidine der Formel (I) erhält, wenn man
a) Nitromethylen-Derivate der Formel (II) 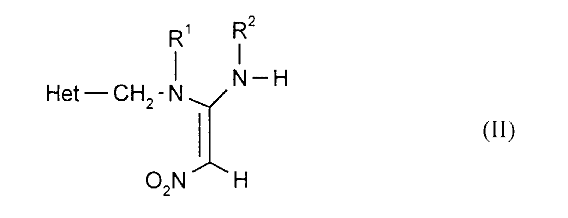 in welcher
   - Het, R¹ und R²: die oben angegebenen Bedeutungen haben,
   mit Aminen der Fomel (III),

   H₂N-A-R³ (III)

   in welcher
   - A und R³: die oben angegebene Bedeutung haben,
   in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt, oder
b) für den Fall, daß in Formel (I) R³ für den Rest 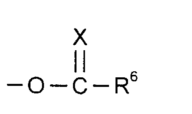 oder den Rest 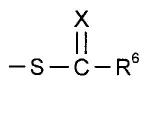 steht,
   Nitromethylen-Derivate der Formeln (IV) oder (IVa) 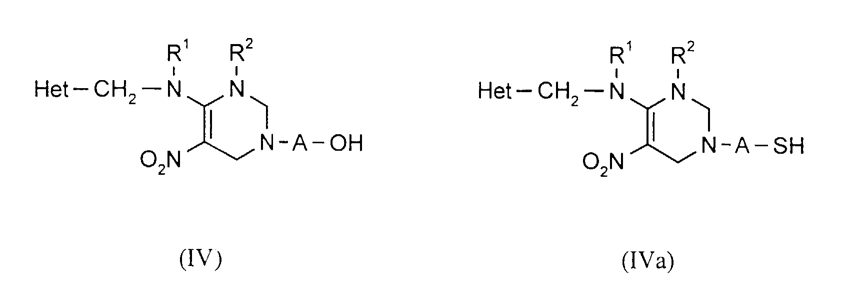 in welchen
   - Het, R¹, R² und A: die oben angegebenen Bedeutungen haben,
   mit Verbindungen der Formel (V) 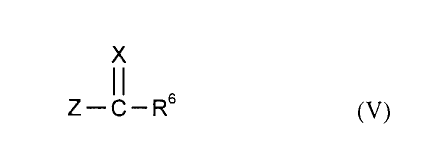 in welcher
   - Z: für eine Abgangsgruppe wie Halogen oder den Rest 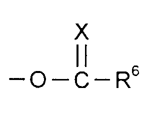 steht, und
   - X und R⁶: die oben angegebene Bedeutung haben,
   oder mit Verbindungen der Formel (VI)

   XCN-R⁹ (VI)

   in welcher
   - X: für 0 oder S steht und
   - R⁹: für einen der folgenden bei R⁶ genannten Reste Alkyl, Aryl oder Aralkyl steht,
   gegebenenfalls in Gegenwart eines Säurebindemittels und gegebenenfalls in Gegenwart von Verdünnungsmitteln und/oder Katalysatoren umsetzt, oder
c) für den Fall, daß in Formel (I) R¹ und R² gemeinsam mit den angrenzenden Atomen keinen Ring schließen, man Nitromethylen-Derivate der Formel (A) 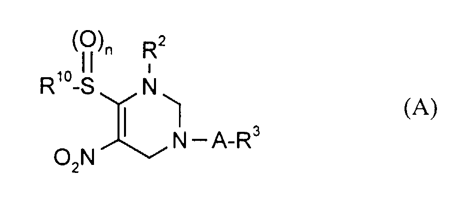 in welcher
   - R¹⁰: für C₁-C₄-Alkyl oder Phenyl steht,
   - n: die Werte 0, 1 oder 2 hat,
   - R², R³, A: die oben angegebenen Bedeutungen haben,
   mit Aminen der Formel (B)

   Het-CH₂-NHR¹ (B)

   in welcher
   - Het und R¹: die oben angegebene Bedeutung haben,
   gegebenenfalls in Gegenwart eines Säurebindemittels und gegebenenfalls von Verdünnungsmitteln und/oder Katalysatoren umsetzt.

Gegenstand der vorliegenden Erfindung sind auch die Nitromethylenderivate der Formeln (IVb), (IVc) und (IVd) 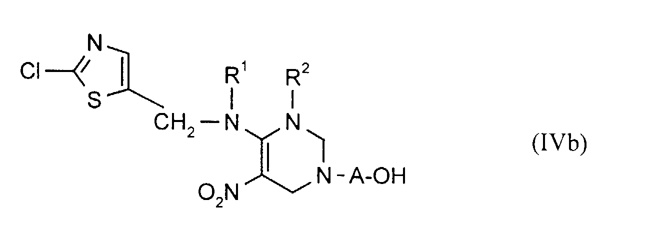 in welcher
- R¹, R² und A: die oben angegebene Bedeutung haben, 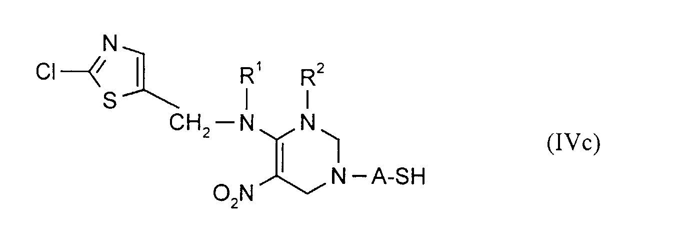
in welcher
- R¹, R² und A: die oben angegebene Bedeutung haben, 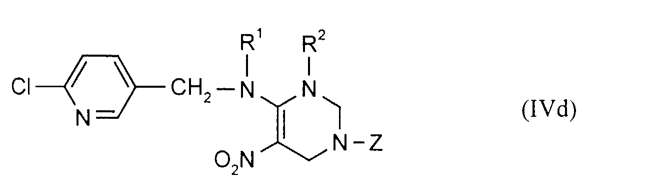
in welcher
- R¹ und R²: die oben angegebene Bedeutung haben und
- Z: für 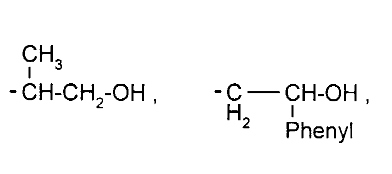 -(CH₂)₄-OH steht.

Bevorzugt sind diejenigen Verbindungen der Formel (I), in welcher
- Het: für 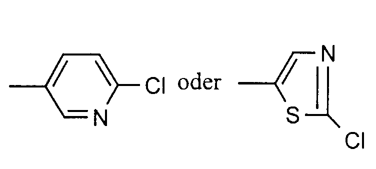 steht und
- R¹: für Methyl oder Ethyl steht,
- R²: für Methyl oder Ethylen steht,
- R¹ und R²: gemeinsam mit den angrenzenden Atomen für einen gesättigten 5- oder 6-gliedrigen Ring, insbesondere für den folgenden Ring 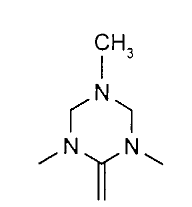 stehen,
- A: für Ethylen, Propylen, Butylen, die gegebenenfalls durch Halogen insbesondere Fluor oder Chlor oder Phenyl substituiert sein können steht, ferner steht A für Cyclohexylen
- R³: steht für einen der Reste 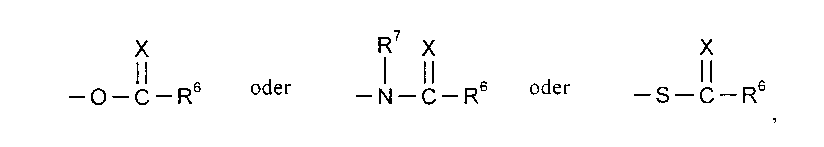 wobei
R⁶ für C₁₋₆-Alkyl wie Methyl, Ethyl, n-Propyl, i-Propyl, s-Butyl. t-Butyl, n-Hexyl, Methoxy, Ethoxy, n- oder i-Propoxy, Methylamino, Dimethylamino, Ethylamino, Diethylamino, Ethylmethylamino, n-Propylamino, Di-(n-propyl)amino, Methyl(n-Propyl)amino, Ethyl(n-propyl)amino, Methylisopropylamino, Ethylisopropylamino, Isopropyl(n-propyl)amino, Cyclopentyl, Cyclohexyl, Cyclopentylmethyl, Cyclopentylethyl, Cyclopentyl-n-propyl, Cyclopentylisopropyl, Cyclohexylmethyl, Cyclohexylethyl, Cyclohexyl-n-propyl, Cylclohexylisopropyl, Phenylmethyl, Phenylethyl, Phenyl-n-propyl und/oder Phenylisopropyl, Phenyl, Pyridyl, Imidazolyl, Thiazolyl, Oxazolyl, Phenoxy, C₁₋₄-Alkylthio wie Methylthio, Ethylthio, n- oder i-Propylthio, Phenylthio steht, wobei die Reste gegebenenfalls durch C₁₋₄-Alkyl, Halogen wie Fluor oder Chlor oder CN substituiert sein können,
R⁷ für Methyl oder Wasserstoff steht und
X für Schwefel oder Sauerstoff steht,
mit der Maßgabe, daß R⁶ nicht für C₁₋₆-Alkyl steht, wenn X für Sauerstoff steht und R⁷ für Wasserstoff steht und A für gegebenenfalls durch Halogen, OH, CN, Amino, Mono- oder DiC₁₋₄-alkylamino substituiertes geradkettiges oder verzweigtes C₂₋₆-Alkyl steht und R¹ und R² gemeinsam mit den angrenzenden Atomen einen unsubstituierten gesättigten 5- oder 6-gliedrigen Ring bilden.

Beispiele für die erfindungsgemäßen Verbindungen der Formel (I) sind in den folgenden Tabellen 1 bis 128 aufgeführt.

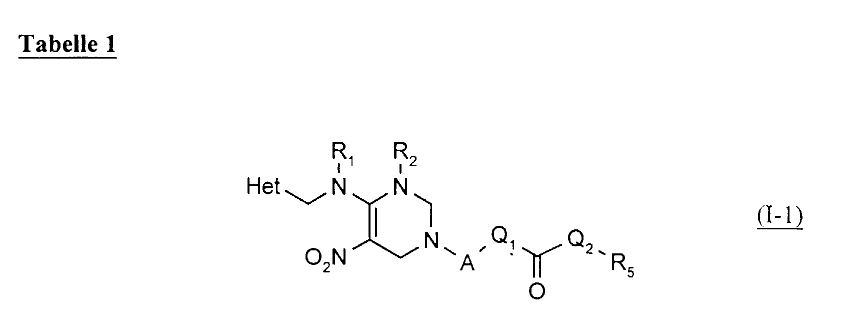

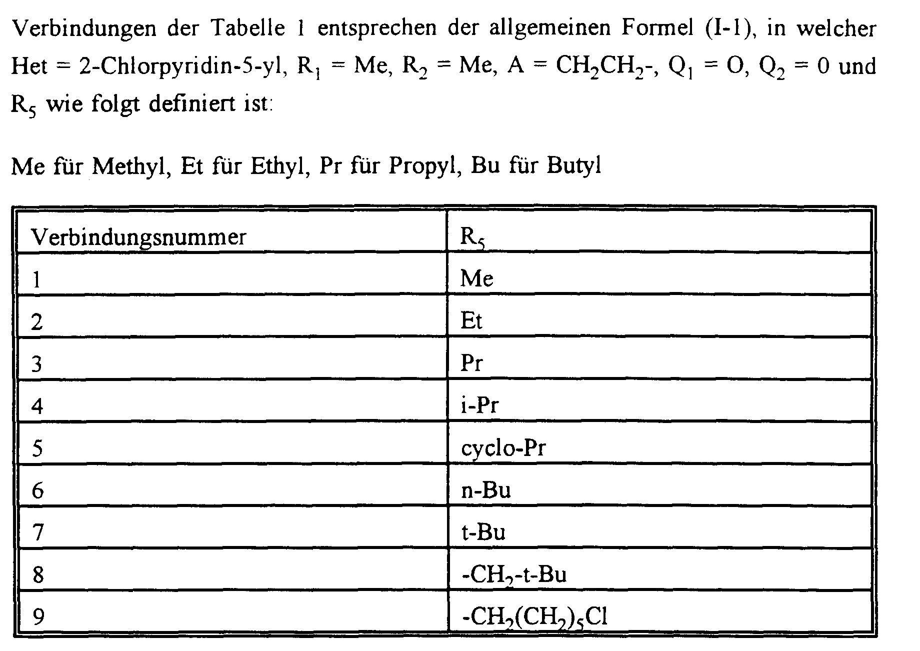

### Tabelle 2

Tabelle 2 enthält die Verbindungen der allgemeinen Formel (I-1), in welcher Het = 2-Chlorpyridin-5-yl, R₁ = Me, R₂ = Me, A = -CH₂CH₂-, Q₁ = O, Q₂ = NH und R₅ wie in Tabelle 1 aufgelistet definiert ist.

### Tabelle 3

Tabelle 3 enthält die Verbindungen der allgemeinen Formel (I-1), in welcher Het = 2-Chlorpyridin-5-yl, R₁ = Me, R₂ = Me, A = -CH₂CH₂-, Q₁ = NH, Q₂ = O und R₅ wie in Tabelle 1 aufgelistet definiert ist.

### Tabelle 4

Tabelle 4 enthält die Verbindungen der allgemeinen Formel (I-1), in welcher Het = 2-Chlorpyridin-5-yl, R₁ = Me, R₂ = Me, A = -CH₂CH₂-, Q₁ = NH, Q₂ = NH und R₅ wie in Tabelle 1 aufgelistet definiert ist.

### Tabelle 5

Tabelle 5 enthält die Verbindungen der allgemeinen Formel (I-1), in welcher Het = 2-Chlorpyridin-5-yl, R₁ = Me, R₂ = Me, A = -CH₂CH₂CH₂-, Q₁ = O, Q₂ = O und R₅ wie in Tabelle 1 aufgelistet definiert ist.

### Tabelle 6

Tabelle 6 enthält die Verbindungen der allgemeinen Formel (I-1), in welcher Het = 2-Chlorpyridin-5-yl, R₁ = Me, R₂ = Me, A = -CH₂CH₂CH₂-, Q₁ = O, Q₂ = NH und R₅ wie in Tabelle 1 aufgelistet definiert ist.

### Tabelle 7

Tabelle 7 enthält die Verbindungen der allgemeinen Formel (I-1), in welcher Het = 2-Chlorpyridin-5-yl, R₁ = Me, R₂ = Me, A= -CH₂CH₂CH₂-, Q₁ = NH, Q₂ = O und R₅ wie in Tabelle 1 aufgelistet definiert ist.

### Tabelle 8

Tabelle 8 enthält die Verbindungen der allgemeinen Formel (I-1), in welcher Het = 2-Chlorpyridin-5-yl, R₁ = Me, R₂ = Me, A = -CH₂CH₂CH₂-, Q₁ = NH, Q₂ = NH und R₅ wie in Tabelle 1 aufgelistet definiert ist.

### Tabelle 9

Tabelle 9 enthält die Verbindungen der allgemeinen Formel (I-1), in welcher Het = 2-Chlorpyridin-5-yl, R₁ = Me, R₂ = Me, A = -CH(CH₃)CH₂-, Q₁ = O, Q₂ = O und R₅ wie in Tabelle 1 aufgelistet definiert ist.

### Tabelle 10

Tabelle 10 enthält die Verbindungen der allgemeinen Formel (I-1), in welcher Het= 2-Chlorpyridin-5-yl, R₁ = Me, R₂ = Me, A= -CH(CH₃)CH₂-, Q₁ = O, Q₂ = NH und R₅ wie in Tabelle 1 aufgelistet definiert ist.

### Tabelle 11

Tabelle 11 enthält die Verbindungen der allgemeinen Formel (I-1), in welcher Het = 2-Chlorpyridin-5-yl, R₁ = Me, R₂ = Me, A = -CH(CH₃)CH₂-, Q₁ = NH, Q₂ = O und R₅ wie in Tabelle 1 aufgelistet definiert ist.

### Tabelle 12

Tabelle 12 enthält die Verbindungen der allgemeinen Formel (I-1), in welcher Het = 2-Chlorpyridin-5-yl, R₁ = Me, R₂ = Me, A = -CH(CH₃)CH₂-, Q₁ = NH, Q₂ = NH und R₅ wie in Tabelle 1 aufgelistet definiert ist.

### Tabelle 13

Tabelle 13 enthält die Verbindungen der allgemeinen Formel (I-1), in welcher Het = 2-Chlorpyridin-5-yl, R₁ = Me, R₂ = Me, A = -CH₂CH(CH₃)-, Q₁ = O, Q₂ = O und R₅ wie in Tabelle 1 aufgelistet definiert ist.

### Tabelle 14

Tabelle 14 enthält die Verbindungen der allgemeinen Formel (I-1), in welcher Het= 2-Chlorpyridin-5-yl, R₁ = Me, R₂ = Me, A= -CH₂CH(CH₃)-, Q₁ = O, Q₂ = NH und R₅ wie in Tabelle 1 aufgelistet definiert ist.

### Tabelle 15

Tabelle 15 enthält die Verbindungen der allgemeinen Formel (I-1), in welcher Het = 2-Chlorpyridin-5-yl, R₁ = Me, R₂ = Me, A = -CH₂CH(CH₃)-, Q₁ = NH, Q₂ = O und R₅ wie in Tabelle 1 aufgelistet definiert ist.

### Tabelle 16

Tabelle 16 enthält die Verbindungen der allgemeinen Formel (I-1), in welcher Het = 2-Chlorpyridin-5-yl, R₁ = Me, R₂ = Me, A = -CH₂CH(CH₃)-, Q₁ = NH, Q₂ = NH und R₅ wie in Tabelle 1 aufgelistet definiert ist.

### Tabelle 17 bis 32

Jede der Tabellen 17 bis 32 enthält die Verbindungen der allgemeinen Formel (I-1), in welcher
A, Q₁, Q₂ und R₅ für die Bedeutungen der Tabellen 1 bis 16 stehen,
Het = 2-Chlorpyridin-5-yl, R₁ = Me und R₂ = Et ist.

### Tabelle 33 bis 48

Jede der Tabellen 33 bis 48 enthält die Verbindungen der allgemeinen Formel (I-1), in welcher
A, Q₁, Q₂ und R₅ für die Bedeutungen der Tabellen 1 bis 16 stehen,
Het = 2-Chlorpyridin-5-yl, R₁ = Et und R₂ = Me ist.

### Tabelle 49 bis 64

Jede der Tabellen 49 bis 64 enthält die Verbindungen der allgemeinen Formel (I-1), in welcher
A, Q₁, Q₂ und R₅ für die Bedeutungen der Tabellen 1 bis 16 stehen,
Het = 2-Chlorpyridin-5-yl, R₁ = Et und R₂ = Et ist.

### Tabelle 65 bis 80

Jede der Tabellen 65 bis 80 enthält die Verbindungen der allgemeinen Formel (I-1), in welcher
A, Q₁, Q₂ und R₅ für die Bedeutungen der Tabellen 1 bis 16 stehen,
Het = 2-Chlorthiazol-5-yl, R₁ = Me und R₂ = Me ist.

### Tabelle 81 bis 96

Jede der Tabellen 81 bis 96 enthält die Verbindungen der allgemeinen Formel (I-1), in welcher
A, Q₁, Q₂ und R₅ für die Bedeutungen der Tabellen 1 bis 16 stehen,
Het = 2-Chlorthiazol-5-yl, R₁ = Me und R₂ = Et ist.

### Tabelle 97 bis 112

Jede der Tabellen 97 bis 112 enthält die Verbindungen der allgemeinen Formel (I-1), in welcher
A, Q₁, Q₂ und R₅ für die Bedeutungen der Tabellen 1 bis 16 stehen,
Het = 2-Chlorthiazol-5-yl, R₁ = Et und R₂ = Me ist.

### Tabelle 113 bis 128

Jede der Tabellen 113 bis 128 enthält die Verbindungen der allgemeinen Formel (I-1), in welcher
A, Q₁, Q₂ und R₅ für die Bedeutungen der Tabellen 1 bis 16 stehen,
Het = 2-Chlorthiazol-5-yl, R₁ = Et und R₂ = Et ist.

Die für die Verbindung der allgemeinen Formel (I) angegebenen allgemeinen und bevorzugten Restedefinitionen gelten auch für die Verbindungen der übrigen allgemeinen Formeln (Zwischen- bzw. Vorprodukte) entsprechend.

Bevorzugte erfindungsgemäße Verbindungen sind auch Additionsprodukte aus Säuren und den substituierten 1,2,3,4-Tetrahydro-5-nitro-pyrimidinen der Formel (I).

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Schwefelsäure, Salpetersäure, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure, Trichloressigsäure, Phthalsäure, Naphthalinsulfonsäure, Nikotinsäure, Citronensäure und Ascorbinsäure.

Verwendet man beispielsweise für das erfindungsgemäße Verfahren a) 3-(2-Chlorpyridin-5-yl-methyl)-2-nitro-methylen-imidazolidin, 4-t-Butylcarbamoyloxy-cyclohexylamin und mindestens die zweifache molare Menge Formaldehyd als Ausgangsstoffe, so kann die entsprechende Reaktion durch das folgende Formelschema wiedergegeben werden:

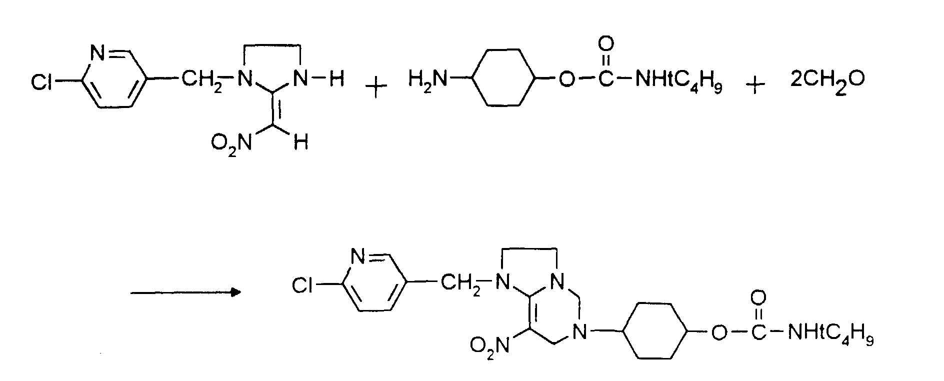

Die beim erfindungsgemäßen Verfahren als Ausgangsstoffe zu verwendenden Verbindungen der Formel (II) sind bekannt und/oder lassen sich nach bekannten Methoden herstellen (vergl. z. B. DE-OS 2 514 402, EP-OS 136 636, EP-OS 154 178 und EP-OS 163 855).

Die beim erfindungsgemäßen Verfahren außerdem als Ausgangsstoffe zu verwendenden Amine der Formel (III) sind allgemein bekannte Verbindungen der organischen Chemie.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei Wasser und für die Umsetzung inerte organische Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, n-Propanol und Isopropanol. Bevorzugt werden Gemische aus Alkoholen und Wasser eingesetzt.

Das erfindungsgemäße Verfahren wird gegebenenfalls in Gegenwart von sauren, nicht oxidierenden Katalysatoren durchgeführt. Besonders bewährt haben sich Halogenwasserstoffsäuren wie Salzsäure und Bromwasserstoffsäure, Phosphorsäure, niedere Carbonsäuren wie Essigsäure und Propionsäure.

Die Reaktionstemperaturen können bei dem erfindungsgemäßen Verfahren in einem größeren Bereich variiert werden. Im allgemeinen arbeitet man bei Temperaturen zwischen -20°C und +120°C, vorzugsweise bei Temperaturen zwischen 0°C und +80°C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Nitromethylen-Derivat der Formel (II), 1 bis 1,5 Mol, vorzugsweise 1 bis 1,2 Mol Amin der Formel (III) und 2 bis 4 Mol, vorzugsweise 2 bis 3 Mol Formaldehyd ein.

Die Amine der Formel (III) können gegebenenfalls in Form ihrer wäßrigen Lösungen eingesetzt werden. Für das erfindungsgemäße Verfahren wird Formaldehyd in wäßriger Lösung eingesetzt. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Säureadditions-Salze der Verbindungen der Formel (I) können in einfacher Weise nach üblichen Salzbildungsmethoden, z. B. durch Lösung einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Verwendet man beispielsweise für das erfindungsgemäße Verfahren b) 6,7-Dihydro-6-(2-hydroxyethyl)-8-nitro-(5H)-3-(2-chlorpyridin-5-yl-methyl)-imidazolidino-(2,3f)-pyrimidin und Chlorameisensäurethiopropylester als Ausgangsstoffe läßt sich das Verfahren durch folgendes Formelschema darstellen:

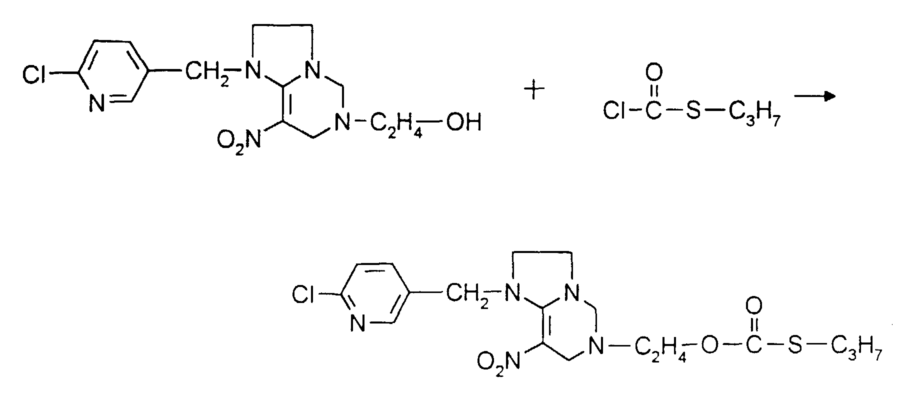

Die Verbindungen der Formel (IV) sind an sich bekannt oder lassen sich analog zu bekannten Verfahren herstellen (vgl. EP-OS 375 613, 316 843, 407 594). Die Verbindungen der Formel (V) sind bekannt.

Die Reaktion der Verbindungen der Formel (IV) und (V) wird vorzugsweise unter Verwendung vom Verdünnungsmittel und in Gegenwart eines basischen Reaktionshilfsmittel durchgeführt.

Als Verdünnungsmittel zur Durchführung des erfindungsgemäßen Verfahrens 2 b) kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Ether wie Ethylpropylether, Methyl-tert.- butylether, n-Butylether, Di-n-butylether, Diisobutylether, Diisoamylether, Diisopropylether, Anisol, Phenetol, Cyclohexylmethyl ether, Diethylether, Ethylenglycoldimethylether, Tetrahydrofuran, Dioxan, Dichlordiethylether, Nitrokohlenwassersstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische, cycloaliphatische oder aromatische Kohlenwasserstoffe wie Heptan, Hexan, Nonan, Cymol. Benzinfraktionen innerhalb eines Siedepunktintervalles von 70°C bis 190°C, Cyclohexan, Methylcyclohexan, Petrolether, Ligroin, Octan, Benzol, Toluol, Xylol; Ester wie Ethylacetat, Isobutylacetat; N,N-Dimethylformamid, N-Methyl-pyrrolidon; Ketone wie Aceton, Methylethylketon. Auch Gemische der genannten Lösungs- und Verdünnungsmittel kommen in Betracht.

Bevorzugt sind Ether wie Tetrahydrofuran und Dioxan.

Als basische Reaktionshilfsmittel können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Oxide und Carbonate des Lithiums, Natriums, Kaliums, Magnesiums, Calciums und Bariums, ferner weitere basische Verbindungen wie Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidon, NMethyl-piperidin, N-Methyl-imidazol, N-Methyl-pyrrol, N-Methyl-morpholin, N-Methyl-hexamethylenimin, Pyridin, Chinolin, α-Picolin, β-Picolin, Isochinolin, Pyrimidin, Acridin, N,N,N',N'-Tetra-methylendiamin, N,N,N',N'-Tetraethylendiamin, Chinoxalin, N-Propyl-diisopropylamin, N,N'-Dimethyl-cyclohexylamin, 2,6-Lutidin, 2,4-Lutidin, Triethylendiamin, Diazabicyclooctan (DABCO), Diazabicyclononen (DBN) oder Diazabicycloundecen (DBU).

Vorzugsweise verwendet man Hydroxide des Natirums und Kaliums oder tertiäre Amine, wie beispielsweise Triethylamin, Tribenzylamin oder Trihexylamin.

Das Verfahren 2 b) wird durchgeführt, indem Verbindungn der Formel (IV) und Verbindungen der Formel (V) in Gegenwart eines Überschusses eines der angegebenen basischen Reaktionshilfsmittel in einem der angegebenen Verdünnungsmittel zusammengegeben und gerührt bzw. gegebenenfalls erhitzt werden.

Die Reaktionsdauer beträgt ca. 0,5 bis 48 Stunden. Die Umsetzung erfolgt bei Temperaturen zwischen +10°C und +200°C, bevorzugt zwischen +20°C und +150°C, besonders bevorzugt bei Raumtemperatur oder Siedetemperatur des verwendeten Verdünnungsmittels. Vorzugsweise arbeitet man bei dem Druck, der sich beim Erhitzen auf die erforderliche Reaktionstemperatur unter den Reaktionsbedingungen einstellt.

Zur Durchführung des erfindungsgemäßen Verfahrens setzt man pro Mol an Verbindung der Formel (IV) im allgemeinen 1,0 bis 4,0 Mol, vorzugsweise 1,0 bis 2,0 Mol, an Verbindungen der allgemeinen Formel (V) ein.

Nach vollendeter Umsetzung wird das Reaktionsgemisch gegebenenfalls im Vakuum eingeengt (zu ca. 50 %), der Rückstand mit wäßriger Säure versetzt und die Verbindungen der Formel (I) in an sich bekannter Weise aufgearbeitet. Die anfallenden Produkte lassen sich in üblicher Weise durch Umkristallisieren, Vakuumdestillation oder Säulenchromatographie reinigen (vgl. auch die Herstellungsbeispiele).

Die zur Durchführung von Verfahren 2b) eingesetzten Verbindungen der Formeln (IV), IVa) bzw. (IVb, c, d) lassen sich analog zu dem unter (2a) beschriebenen Verfahren herstellen.

Die in Verfahren (2c) als Ausgangsverbindungen dienenden Verbindungen der Formel A lassen sich herstellen, indem man Verbindungen der Formel C 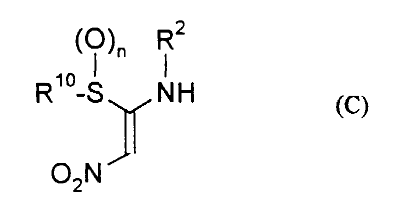 in welcher
- R², R¹⁰ und n: die oben angegebene Bedeutung einnehmen können
wie bei Verfahren (2a) angegeben
in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt, oder
für den Fall, daß in Formel (I) R³ für einen der Reste 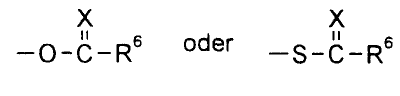 steht,
man Nitromethylen-Derivate der Formel C mit Aminoalkoholen der Formel D

H₂N-A-OH (D)

bzw. Aminothioalkoholen der Formel D'

H₂N-A-SH (D')

in welcher A die obenstehende Bedeutung einnehmen kann wie bei Verfahren 2a angegeben
in Gegenwart von mindestens der zweifachen molaren Menge Formaldehyd, gegebenenfalls in Gegenwart von sauren Katalysatoren und gegebenenfalls in Gegenwart von Verdünnungsmitteln zu Verbindungen der Formel E 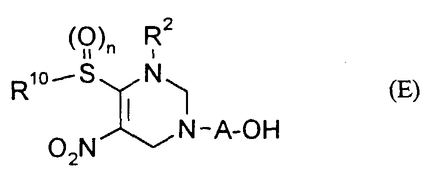 in welcher R², R¹⁰, A und n die oben angegebene Bedeutunghaben,
umsetzt und diese mit Verbindungen der Formel (V) 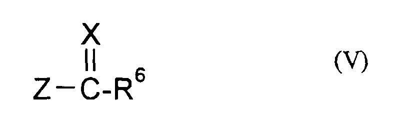 in welcher Z, X und R⁶ die oben angegebene Bedeutung haben,
oder mit Verbindungen der Formel (VI)

XCN-R⁹ (VI)

in welcher X und R⁹ die oben angegebene Bedeutung haben,
gegebenenfalls in Gegenwart eines Säurebindemittels und gegebenenfalls in Gegenwart von Verdünnungsmitteln und/oder Katalysatoren zur Reaktion bringt.

Amine der Formel B sind bekannt (z.B. EP-30 23 389; GB-22 28 003), Nitromethylen-Derivate der Formel C sind bekannt (FR-23 11 003; DE-26 21 092) oder lassen sich zu bekannten Verfahren herstellen.
Die Durchführung von Verfahren (2c) erfolgt nach der bei Verfahren (2b) beschriebenen Methode unter den dort angegebenen Bedingungen.
Die erfindungsgemäßen Verbindungen der Formel (I) können zur Schädlingsbekämpfung eingesetzt werden. Schädlinge sind unerwünschte tierische Schädlinge, insbesondere Insekten. Milben und Nematoden, welche Pflanzen oder höhere Tiere schädigen.
Erfindungsgemäß ist auch ein Verfahren zur Bekämpfung von Schädlingen und Ektoparasiten, dadurch gekennzeichnet, daß man die Verbindungen der Formel (I) auf Schädlinge und/oder ihren Lebensraum (d.h. das Biotop, in dem sie gedeihen) einwirken läßt.
Die erfindungsgemäßen Wirkstoffe eignen sich bei guter Pflanzenverträglichkeit und günstiger Wärmeblütertoxizität zur Bekämpfung von tierischen Schädlingen, vorzugsweise von Arthropoden. insbesondere von Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica. Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp. Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffe können zur Verwendung als Insektizide, Akarizide und Nematizide in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwen-dungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Verbindungen eignen sich auch in besonderer Weise zur Behandlung von vegativem und generativem Vermehrungsmaterial, wie z.B. von Saatgut von Getreide, Mais, Gemüse usw. oder von Zwiebeln, Stecklingen usw.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Zur Herstellung der Schädlingsbekämpfungsmittel können die erfindungsgemäßen Wirkstoffe in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmneben-Formulierungen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Mittel enthalten bevorzugt neben wenigstens einer Verbindung der allgemeinen Formel (I) und gegebenenfalls neben erheblichen Streck- und Hilfsmitteln wenigstens einen oberflächenaktiven Stoff.

Die Wirkstoffe eignen sich bei günstiger Warmblütertoxizität auch zur Bekämpfung von tierischen Schädlingen (Ektoparasiten) wie Arthropoden, vorzugsweise Insekten und Spinnentieren (Ektoparasiten), die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Durch die Bekämpfung der tierischen Schädlinge sollen Krankheiten und deren Übertragung, Todesfälle und Leistungsminderungen (z.B. bei der Produktion von Fleisch, Milch, Wolle, Häuten, Eiern) verhindert werden, so daß durch den Einsatz der Wirkstoffe eine wirtschaftlichere und einfachere Tierhaltung möglich ist bzw. in bestimmten Gebieten erst möglich wird.

Zu den Schädlingen gehören:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp.,
Trichodectes spp., Felicola spp., Damalinea spp., Bovicola spp;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..
Aus der Ordnung der Metastigmata z.B. Hyalomma spp., Rhipicephalus spp., Boophilus spp., Amblyomma spp., Haemophysalis spp., Dermacentor spp., Ixodes spp., Argas spp., Ornithodorus spp., Otobius spp.;
aus der Ordnung der Mesastigmata z.B. Dermanyssus spp., Ornithonyssus spp., Pneumonyssus spp..
Aus der Ordnung der Prostigmata z.B. Cheyletiella spp., Psorergates spp., Myobia spp., Demodex spp., Neotrombicula spp.;
aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp. Lytodites spp., Laminosioptes spp.
Zu den Endoparasiten gehören:

Zu den Nutz- und Zuchttieren gehören Säugetiere wie z.B. Rinder, Pferde, Schafe, Schweine, Ziegel, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff entweder die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

### Lichtschutzmittel sind z.B. Novantisolsäure.

Haftmittel sind z.B. Cellulosederivate, Stärkederivate, Polyacrylate, natürliche Polymere wie Alginate, Gelatine.

### Emulsionen können oral, dermal oder als Injektionen angewendet werden.

### Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.
Fettsäureester wie Ethylstearat, Di-n-butyryl-adipat, Laurinsäurehexylester, Dipropylen-glykolpelargonat,
Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die vorliegende Erfindung betrifft somit auch die Verbindungen der allgemeinen Formel (I) zur Verwendung als Ektoparasitizide sowie die Verwendung der Verbindungen der allgemeinen Formel (I) zur Herstellung eines Mittels zur Bekämpfung von Ektoparasiten.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenyl-imidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoffje kg Körpergewicht pro Tag zur Erzielung wirksamer Ergebnisse zu verabreichen.

Die erfindungsgemäßen Verbindungen werden bevorzugt als Arthropodizie in den Bereichen Pflanzenschutz, Haushalt- und Hygiene sowie im Vorratsschutz, ganz besonders bevorzugt im Pflanzenschutz eingesetzt.

Wo nicht anderes angegeben wird sind alle Prozentangaben Gewichtsprozente.

Die Herstellung der erfindungsgemäßen Verbindungen der allgemeinen Formel (I) soll durch die folgenden Herstellungsbeispiele und die biologische Wirksamkeit durch die folgenden biologischen Beispiele erläutert werden.

### Herstellungsbeispiele

### Beispiel 1

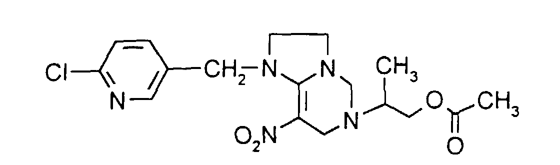

1,8 g (5 mMol) 6,7-Dihydro-6-(1-methyl-2-hydroxy-ethyl)-8-nitro-(5H),3-(2-chlorpyridin-5-yl-methyl)-imidazolidino-[2,3-β-pyrimidin und 0,5 g 4-Pyrrolidino-pyridin werden in 50 ml Pyridin gelöst und langsam mit 1 ml (11 mMol) Acetanhydrid versetzt. Man rührt 4 Stunden bei 60°C, kühlt auf 20°C ab, gießt auf ca. 50 g Eis, extrahiert mit Dichlormethan und destilliert im Wasserstrahlvakuum das Lösungsmittel ab. Anschließend wird der Rückstand an Kieselgel chromatographiert (Laufmittel: Toluol:EtOH = 1:1). Man erhält 1,3 g (66 % der Theorie) 6,7-Dihydro-6-(1-methyl-2-acetyloxy-ethyl)-8-nitro-(5H)-3-(2-chlorpyridin-5-yl-methyl)imidazolidino-[2,3-f]-pyrimidin.

### Beispiel 2

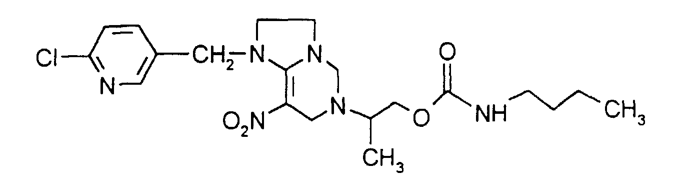

1,8 g(5 mMol) 6,7-Dihydro-6-(1-methyl-2-hydroxy-3ethyl)-8-nitro-(5H)-3-(2-chlorpyridin-5-yl-methyl)-imidazolidino-[2,3-f]-pyrimidin werden in 20 ml Toluol gelöst und bei 20°C mit 1,5 ml (13 mMol) n-Butylisocyanat und 40 mg 1,8-Diazabicyclo[5,4,0]-undec-7-en (1,5-5) (DBU) versetzt. Man erwärmt 24 Stunden auf 50°C, kühlt ab und chromatographiert an Kieselgel (Laufmittel: Toluol:Aceton = 1:1). Man erhält 1,5 g (67 % der Theorie) 6,7-Dihydro-6-(1-methyl-2-butylaminocarbonyloxyethyl)-8-nitro-(5H)-3-(2-chlorpyridin-5-yl-methyl)-imidazolidino-[2,3-f]-pyrimidin.

### Beispiel 3

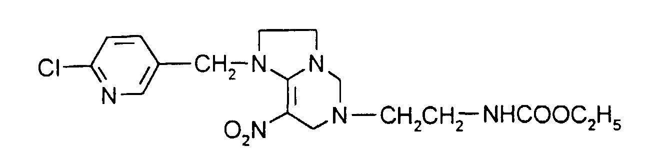

Zu einer Mischung von 6,4 g (0,025 Mol) 3-(2-Chlor-pyridin-5-yl-methyl)-2-nitromethylen-imidazolidin und 3,6 g (0,027 Mol) 1-Amino-2-ethoxycarbonylaminoethan in 25 ml Ethanol werden bei 20°C 4,5 ml einer 30 %igen Formaldehydlösung getropft. Man rührt zwei Stunden unter Rückfluß, kühlt auf 20°C ab und destilliert das Lösungsmittel im Wasserstrahlvakuum weitgehend ab. Anschließend wird an Kieselgel chromatographiert. Man erhält 8 g (82 % der Theorie) 6,7-Dihydro-6-(2-ethoxycarbonylamino-ethyl)-8-nitro-(5H)-3 -(2-chlorpyridin-5-yl-methyl)imidazolidino-[2,3-f]-pyrimidin mit einem Schmelzbereich von 46 bis 48°C.

Analog werden die folgenden Verbindungen der Formel (I) hergestellt.

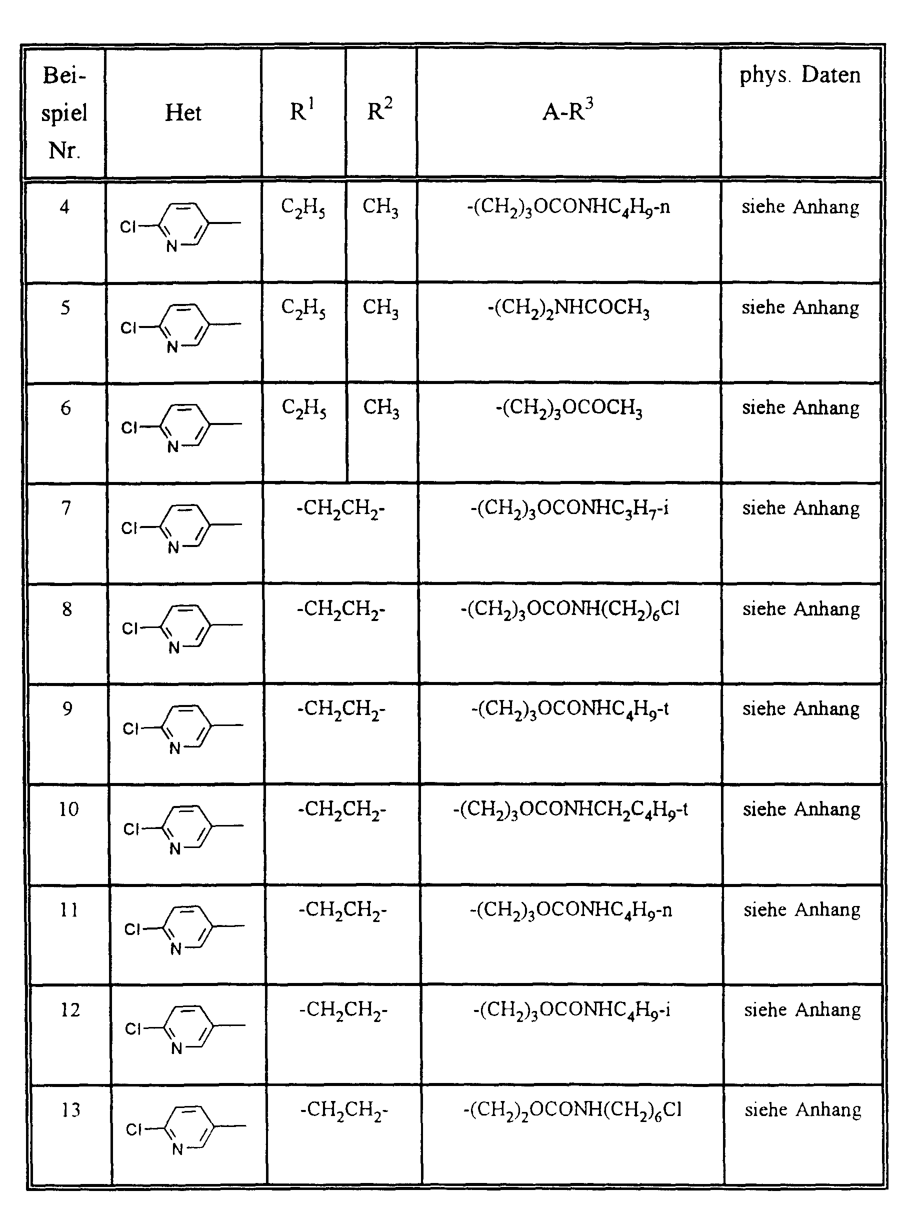

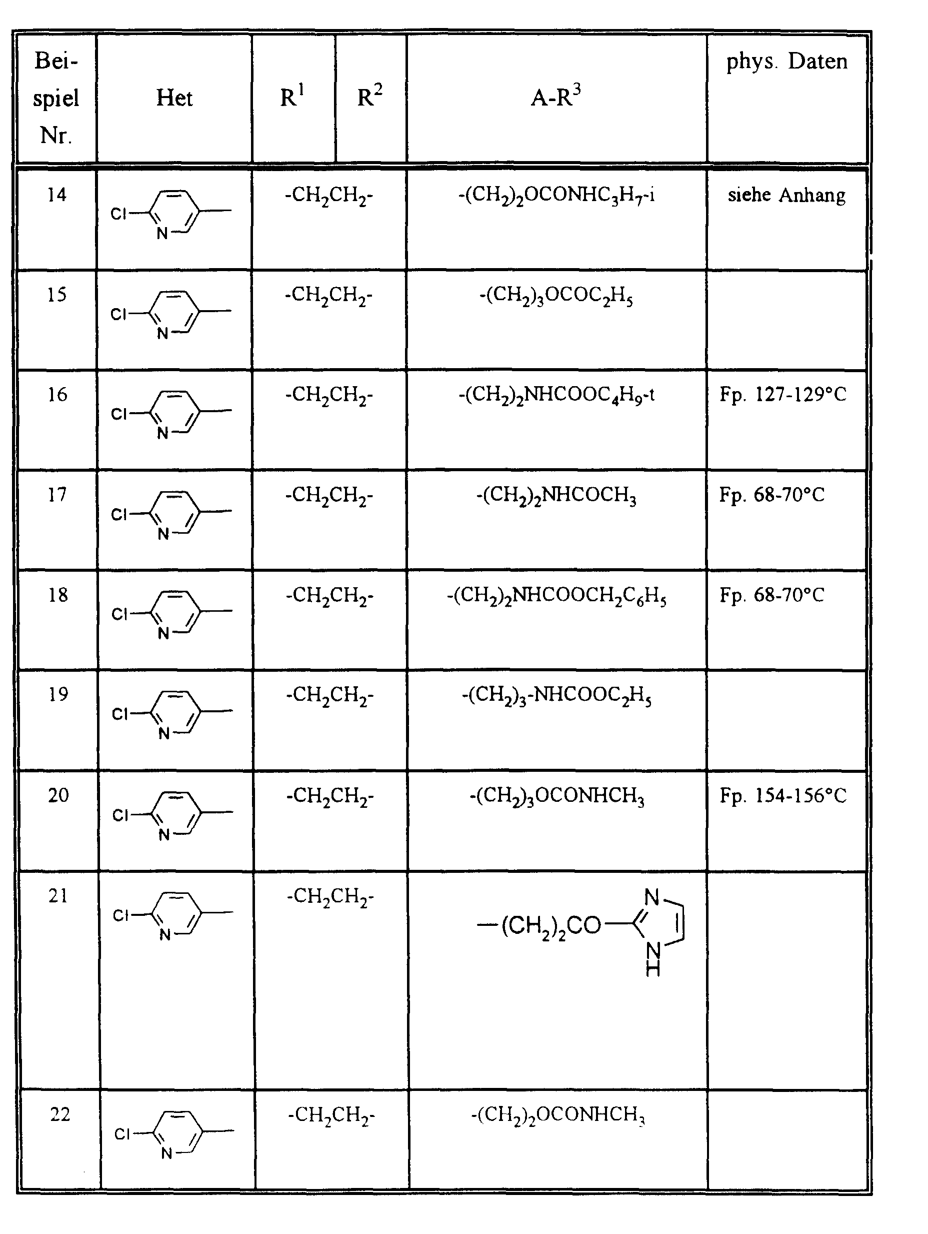

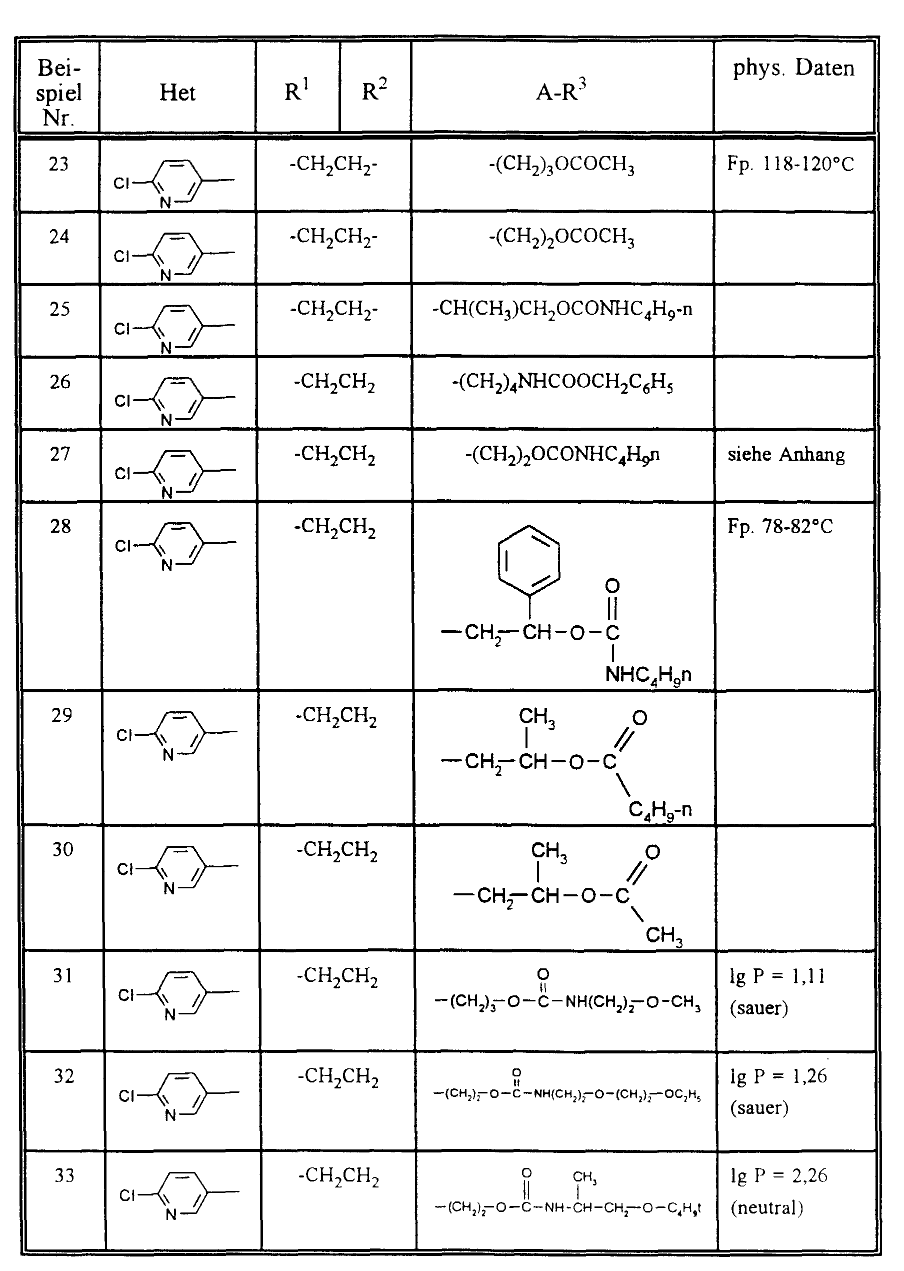

Ferner Verbindungen folgender Formel

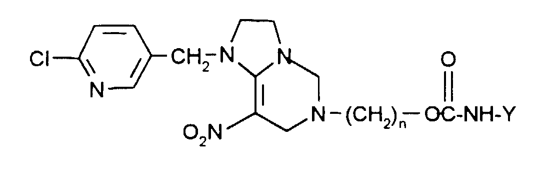

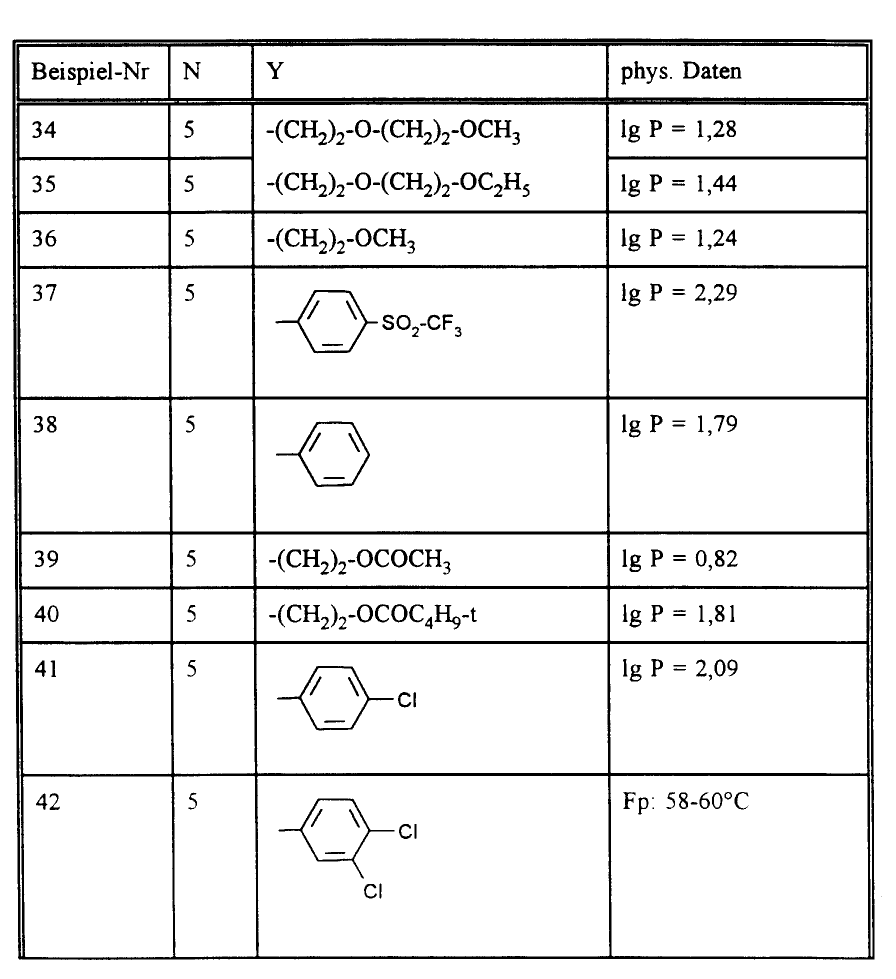

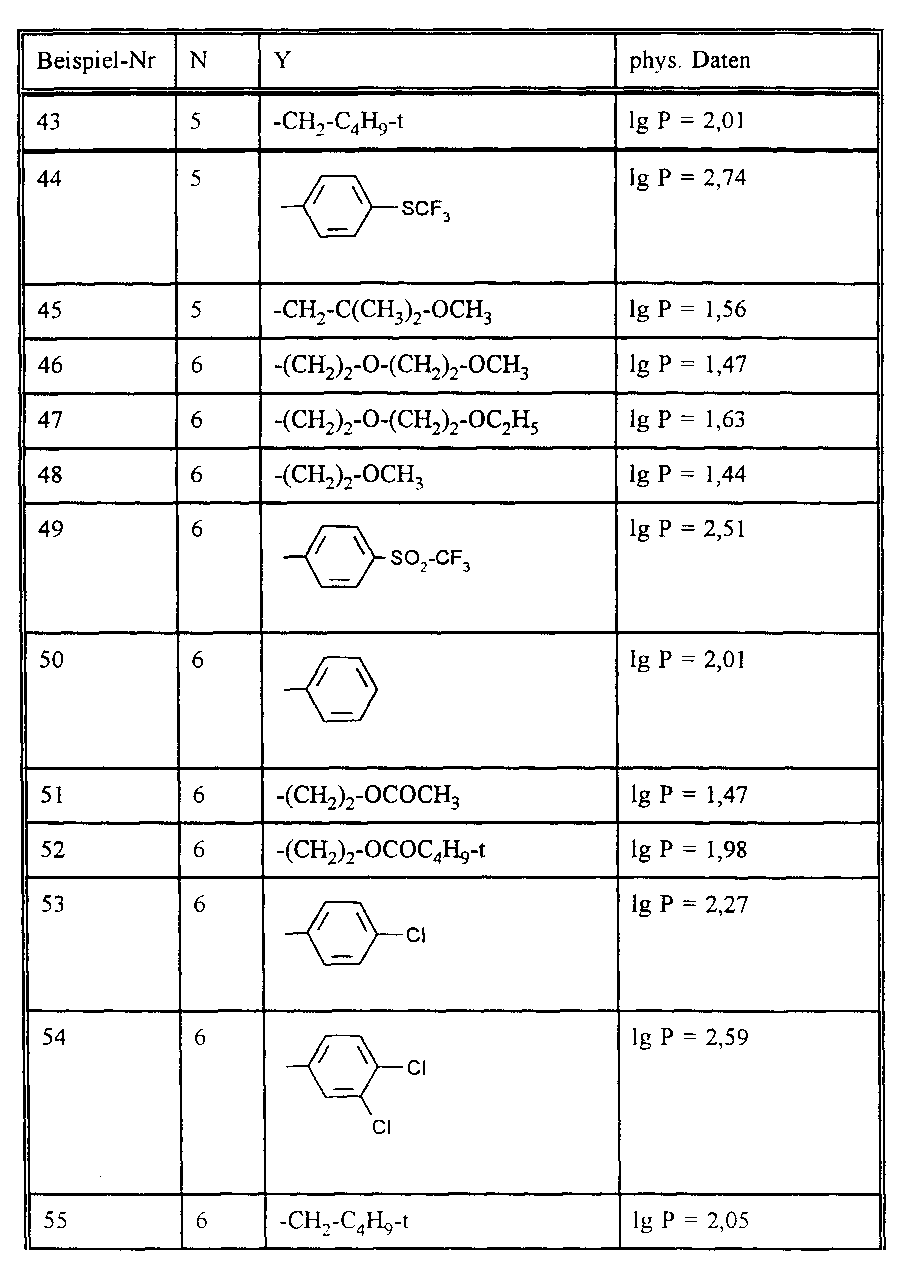

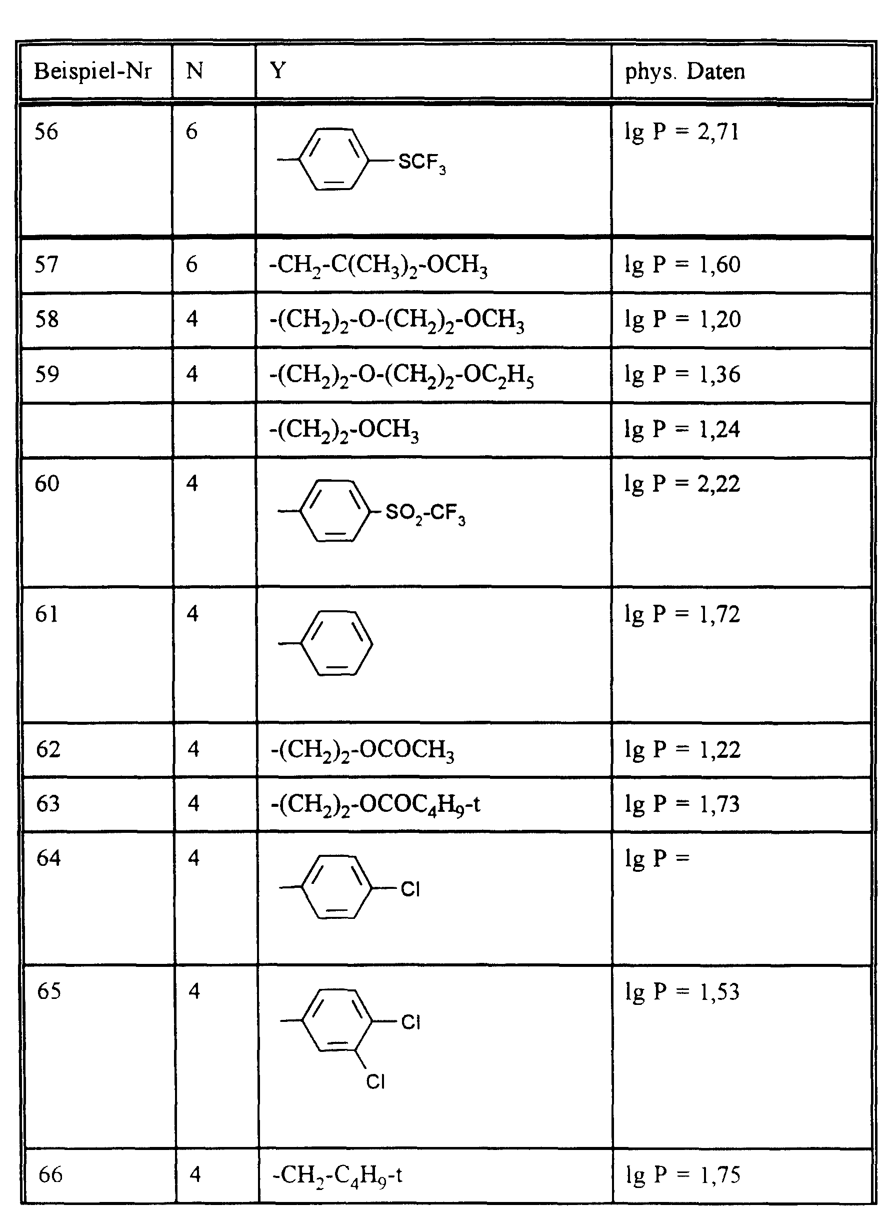

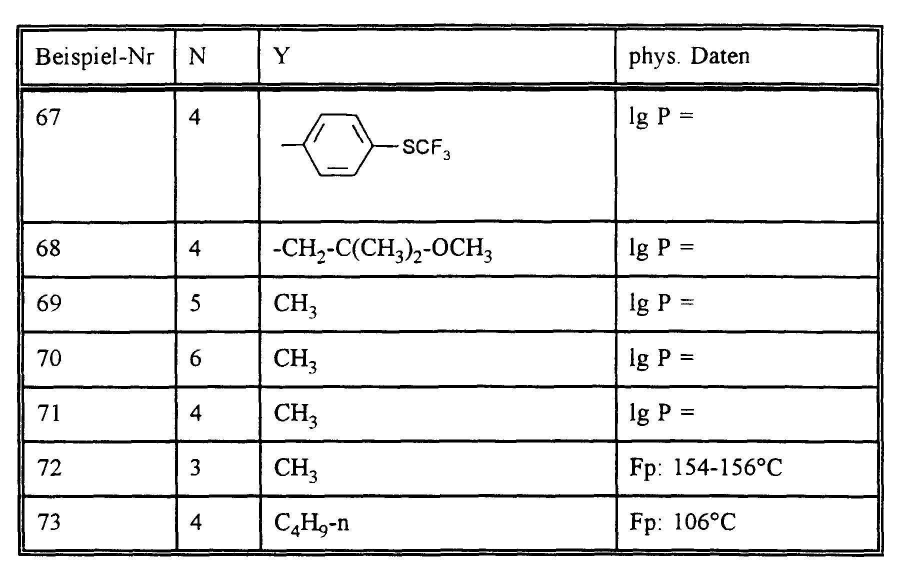

Ferner Verbindungen der Formel I, in welcher die Reste folgende Bedeutung haben:

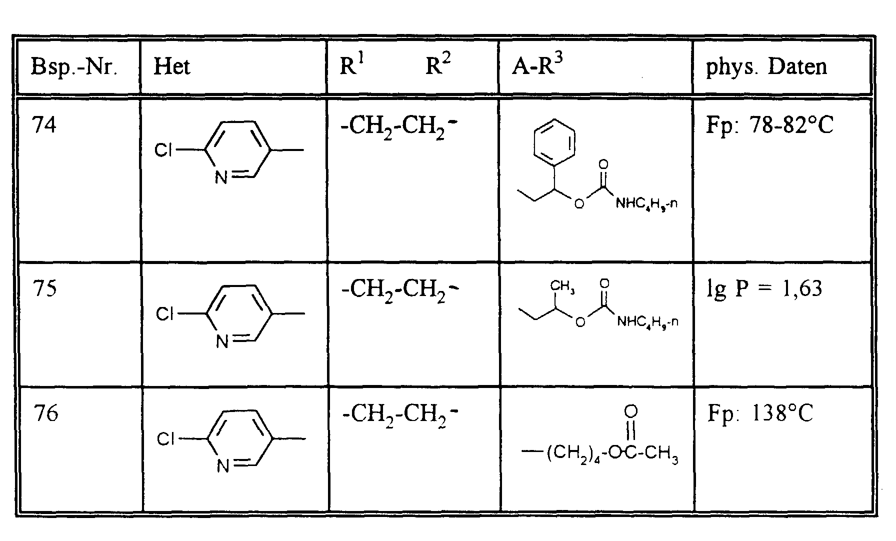

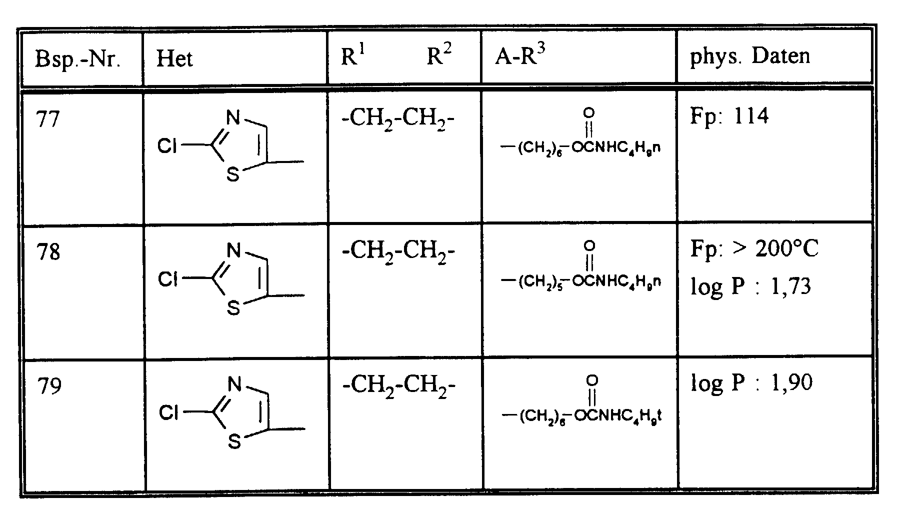

Anhang zu Tabelle 1 :
- Bsp.Nr. 12: ¹H-NMR (DMSO) δ = 0,8 (6h;D), 1,4-1,8 (3H, m), 2,50 (2H, m), 2,82 (2H, m), 3,60 (6H, m), 3,95 (2H, t), 4,05 (2H, s), 4,80 (2H, s), 6,4 (1H, br), 7,50 (1H, d), 7,80 (1H, dd), 8,40 (1H, d).
- Bsp. Nr. 9: ¹H-NMR (DMSO) δ = 1,20 (9H, s), 1,70 (2H, m), 2,50 (2H, m), 3,65 (6H, m), 3,95 (2H, t), 4,08 (2H, s), 4,80 (2H, s), 6,70 (1H, br), 7,50 (1H, d), 7,85 (1H, dd), 8,38 (1H, d).
- Bsp. Nr. 4: NMR (DMSO) δ = 8,34 (d, 1H), 7,77 (dd, 1H), 7,49 (d, 1H), 7,07 (t, 1H), 4,38 (AB, 2H), 3,78 (9, 2H), 0,86 (t, 3H).
- Bsp. Nr. 5: ¹H-NMR (CDCl₃) δ = 8,3 (d, 1H), 7,68 (dd, 1H), 7,33 (d, 1H), 6,07 (br t, 1H), 4,35 (AB, 2H), 3,02 (s, 3H), 2,02 (s, 3H), 1,21 (t, 3H).
- Bsp. Nr. 6: ¹H-NMR (DMSO) δ = 8,33 (d, 1H), 7,77 (dd, 1H), 7,49 (d, 1H), 4,37 (AB, 2H), 3,13 (m, 1H), 1,99 (s, 3H), 1,74 (m, 2H), 1,18 (t, 3H).
- Bsp. Nr. 7: ¹H-NMR (DMSO) δ = 1,05 (6H, d), 2,70 (2H, m), 2,50 (2H, m), 3,5-3,7 (9H, m), 3,95 (2H, t), 4,05 (2H, s), 4,80 (2H, s), 6,85 (1H, br), 7,50 (1H, d), 7,85 (1H, dd), 8,40 (1H, d).
- Bsp. Nr. 8: ¹H-NMR (DMSO) δ = 1,2-1,4 (6H, m), 1,6-1,8 (4H, m), 2,5 (2H, m), 2,95 (2H, m), 3,5-3,7 (6H, m), 3,95 (2H, t), 4,05 (2H, s), 4,80 (2H, s), 7,0 (1H, br), 7,5 (1H, d), 7,85 (1H, dd), 8,38 (1H, d).
- Bsp. Nr. 10: ¹H-NMR (DMSO) δ = 0,8 (9H, s), 1,70 (2H, m), 2,50 (2H, m), 2,80 (2H, s), 3,20 (2H, m), 3,65 (4H, m), 3,95 (2H, t), 4,05 (2H, s), 4,80 (2H, s), 7,0 (1H, br), 7,5 (1H, d), 7,85 (1H, dd), 8,40 (1H, d).
- Bsp. Nr. 11: ¹H-NMR (DMSO) δ = 0,85 (3H, t), 1,1-1,5 (4H, m), 1,70 (2H, m), 2,5 (2H, m), 2,95 (2H, m), 3,65 (6H, m), 3,95 (2H, t), 4,05 (2H, s), 4,80 (2H, s), 6,95 (1H, br), 7,50 (1H, d), 7,85 (1H, dd), 8,38 (1H, d).
- Bsp. Nr. 13: ¹H-NMR (DMSO) δ = 1,2-1,4 (6H, m), 1,6-1,8 (2H, m), 2,70 (2H, m), 2,95 (2H, m), 3,6-3,7 (8H, m), 4,0-4,2 (4H, m), 4,8 (2H, s), 7,0 (H, br), 7,50 (1H, d), 7,85 (1H, dd), 8,4 (1H, d).
- Bsp. Nr. 14: ¹H-NMR (DMSO) δ = 1,0 (6H, d), 2,65 (2H, m), 3,5-3,8 (7H, m), 4,0-4,2 (4H, m), 4,8 (2H, s), 6,95 (1H, br), 7,5 (1H, d), 7,85 (1H, dd), 8,40 (1H, d).
- Bsp. Nr. 27: ¹H-NMR (DMSO) δ = 0,85 (3H, t), 1,2-1,5 (4H, m), 2,70 (2H, m), 2,95 (2H, m), 3,6-3,7 (41, m), 4,0-4,2 (4H, m), 4,3 (2H, s), 4,8 (2H, s), 7,0 (1H, br), 7,5 (1H, d), 7,85 (1H, dd), 8,40 (1H, d).

### Herstellung der Ausgangsverbindungen der Formel (IV)

### Beispiel 1

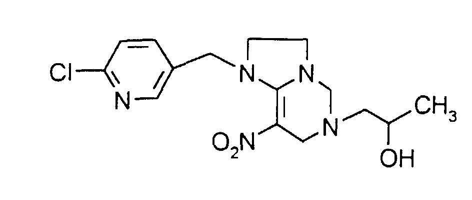

Zu einer Mischung von 6,4 g (0,025 Mol) 3-(2-Chlorpyridin-5-yl-methyl)-2-nitromethylen-imidazolidin und 2,0 g (0,027 Mol) 1-Amino-2-propanol in 25 ml Ethanol werden bei 20°C 4,5 ml einer 30 %igen Formaldehydlösung getropft. Man erhitzt 4 Stunden auf Rückfluß, kühlt dann auf Raumtemperatur ab, destilliert im Wasserstrahlvakuum Ethanol ab und chromatographiert den Rückstand (Laufmittel: Methylenchlorid:Ethanol = 1:1). Man erhält 5,5 g (63 % der Theorie) 6,7-Dihydro-6-(2-hydroxypropyl)-8-nitro-(5H)-3-(2-chlorpyridin-5-yl-methyl)-imidazolidino-[2,3-f]-pyrimidin mit einem Schmelzbereich von 156-158°C.

### Beispiel II-2

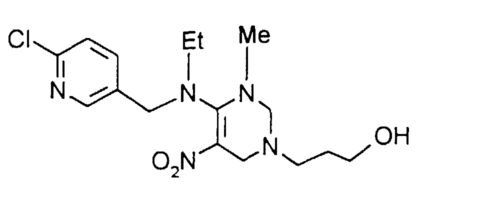

9,6 g (37 mMol) 1-[N-(6-chloro-3-pyridyl)-N-ethyl]amino-1-methylamino-2-nitroethylen (EP 302.389) wurden in 100 ml Ethanol suspendiert. Nach Zugabe von 3,33 g (44 mMol) 3-Amino-1-propanol und 7,6 ml (101 mMol) 37 %ige wäßrige Formaldehydlösung wurde 4 Stunden unter Rückfluß erhitzt. Chromatographie des nach Abdestillieren des Lösunsmittels erhaltenen Rückstands lieferte 8,9 g (63 %) der Titelverbindung als gelbes Öl.

NMR [D-DMSO]: 8,33 (d, 1H), 7,77 (dd, 1H), 7,49 (d, 1H), 4,49-4,24 (m, 3H), 4,11 (q, 2H), 3,37 ('q', 2H), 1,56 (quint, 2H), 1,12 (t, 3H).

Analog werden die Zwischenprodukte der Formel (II) der folgenden Tabelle 2 erhalten:

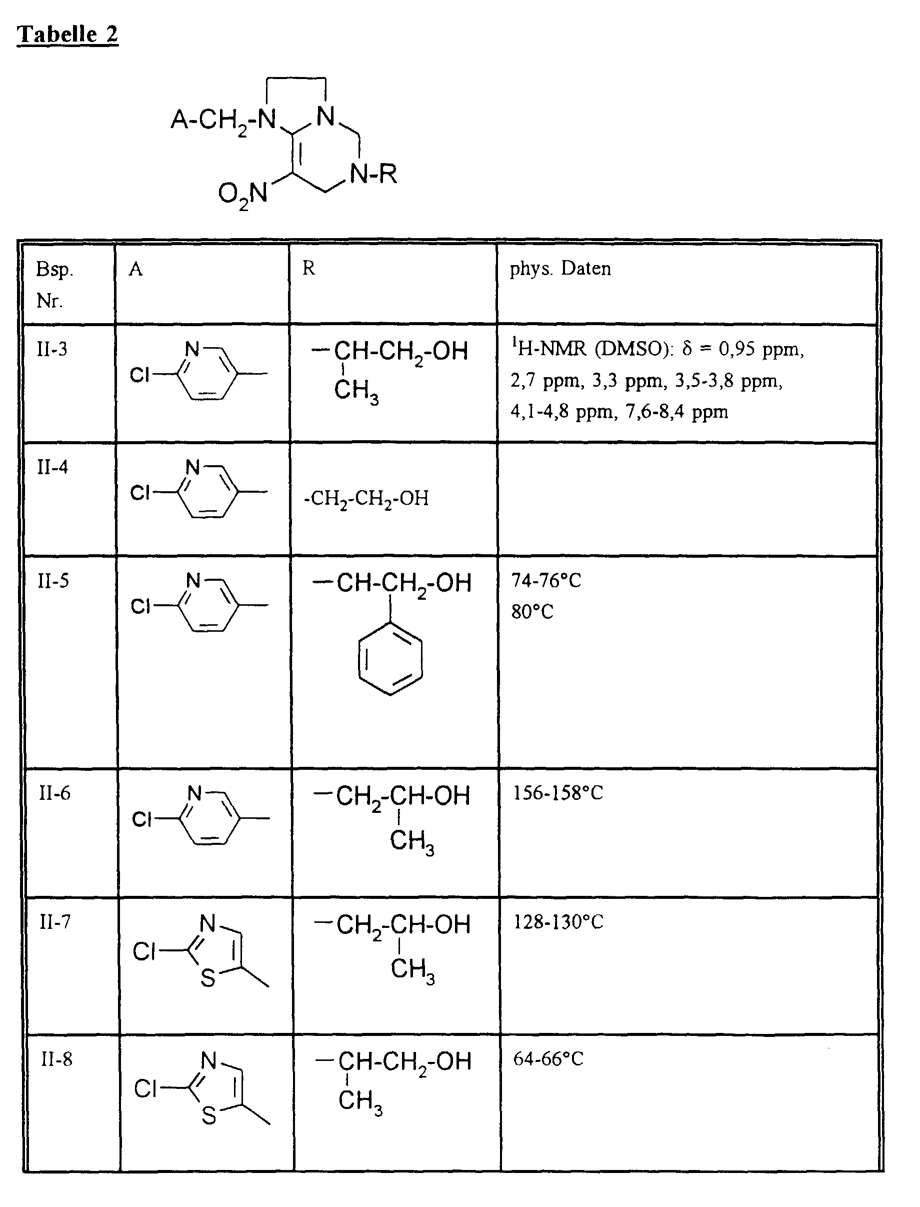

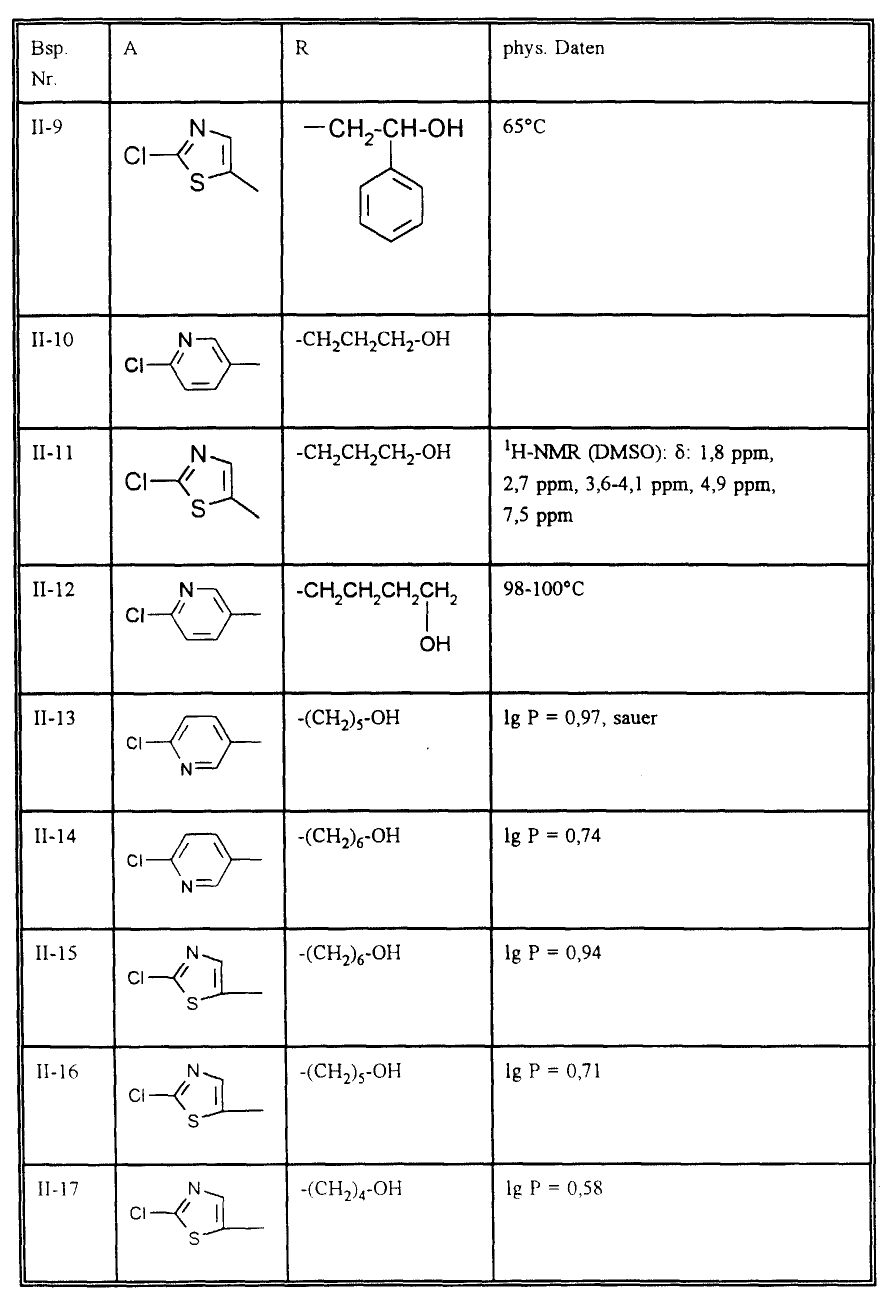

### Beispiel A

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik:

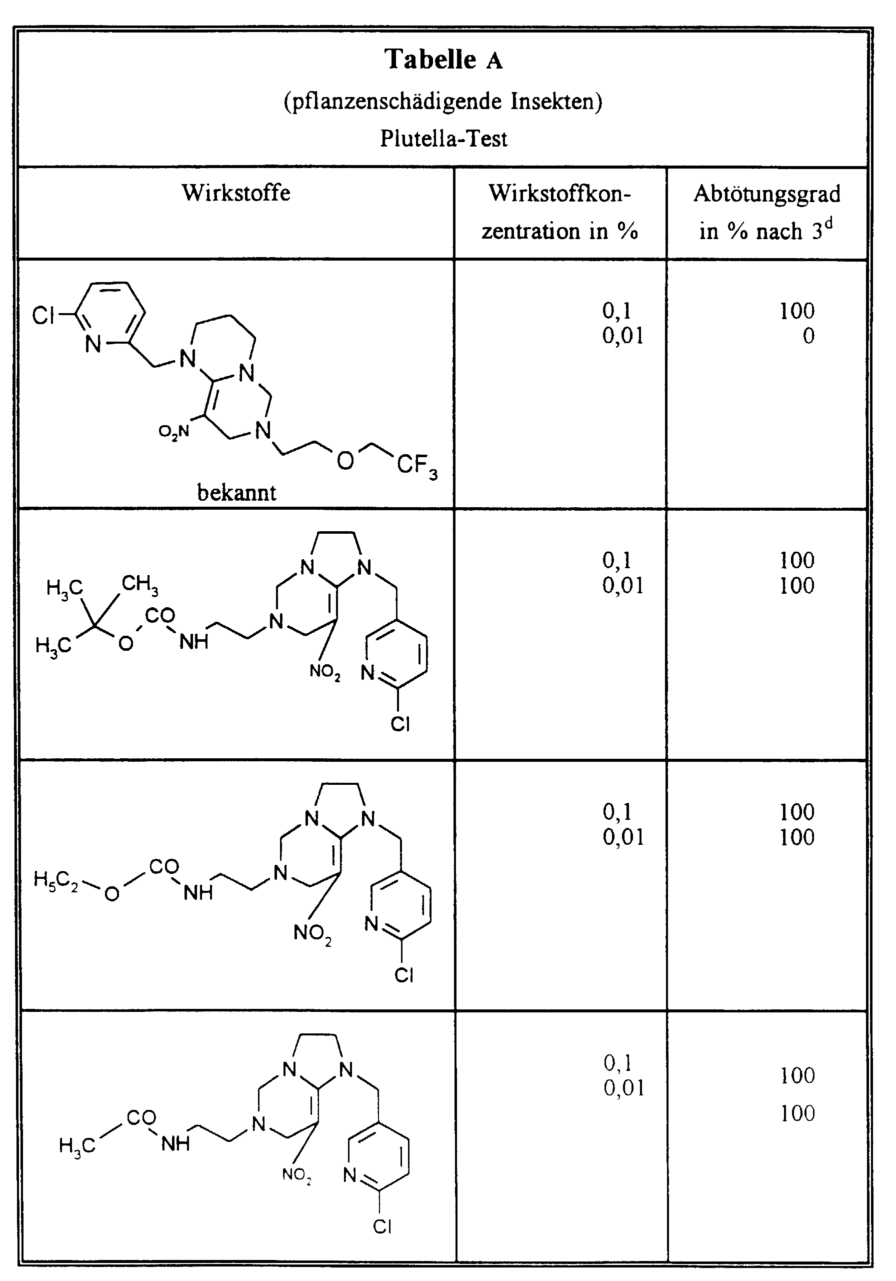

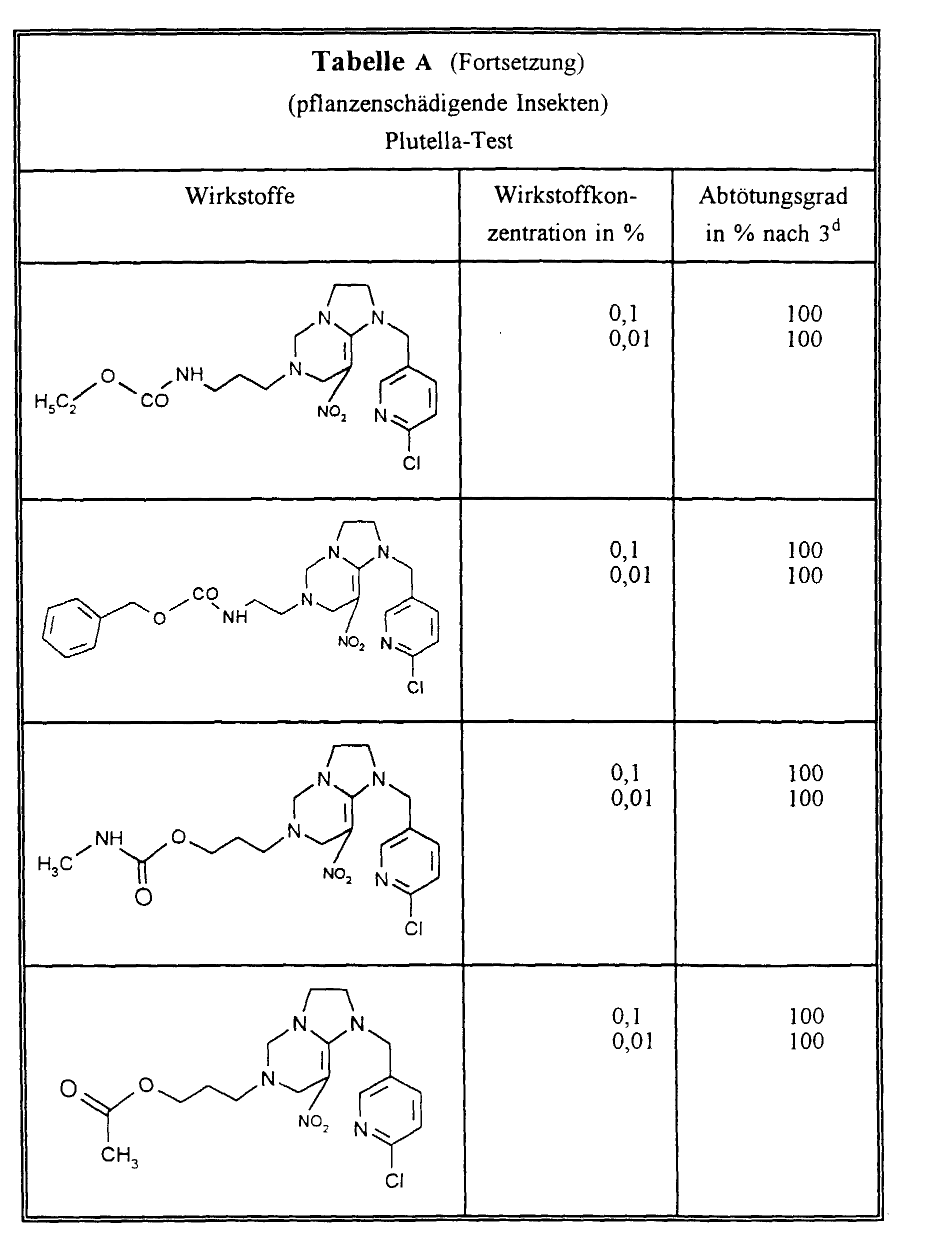

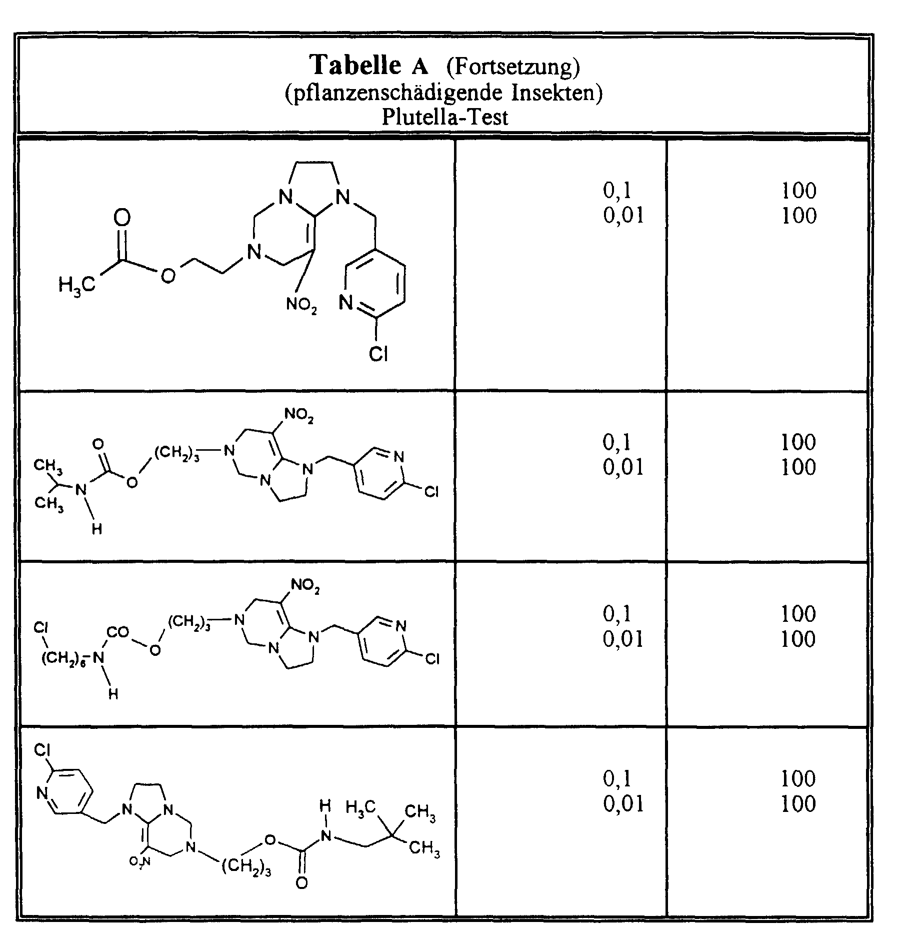

### Beispiel B

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen die folgenden Verbindungen der Herstellungsbeispiele überlegene Wirksamkeit gegenüber dem Stand der Technik:

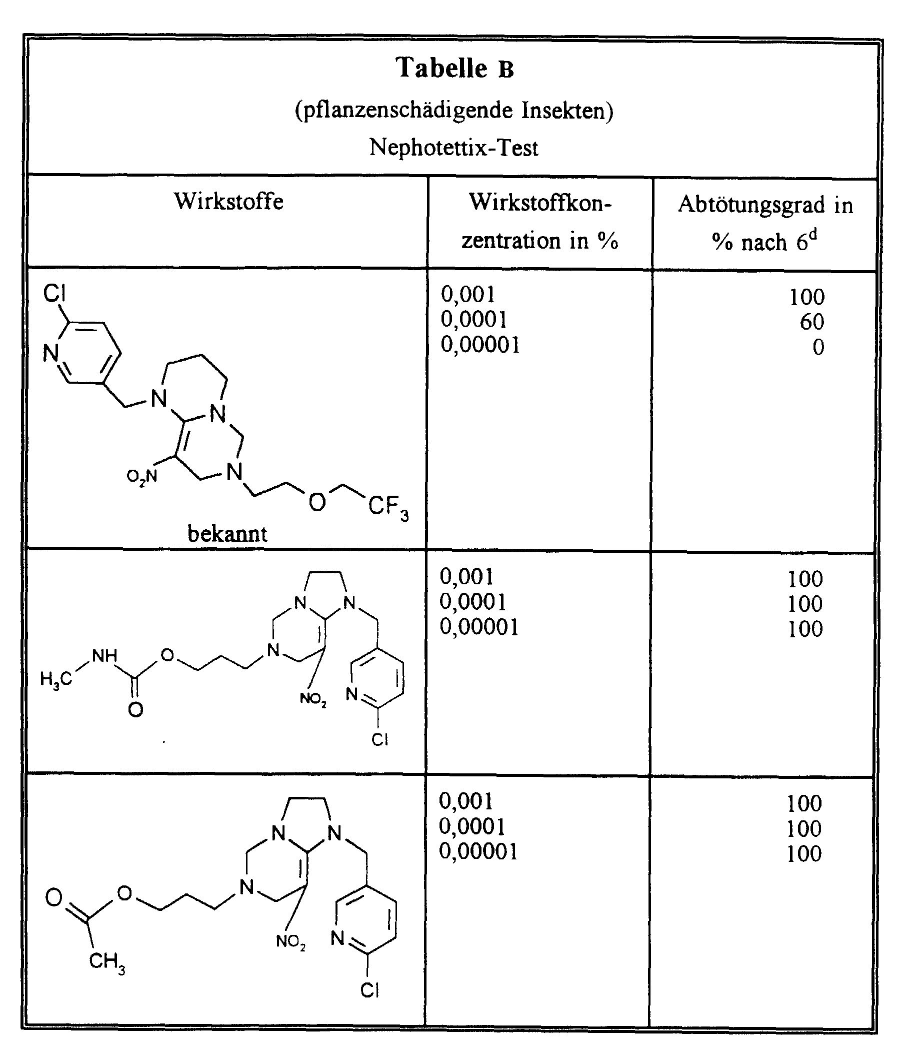

### Beispiel C

### Myzus-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik:

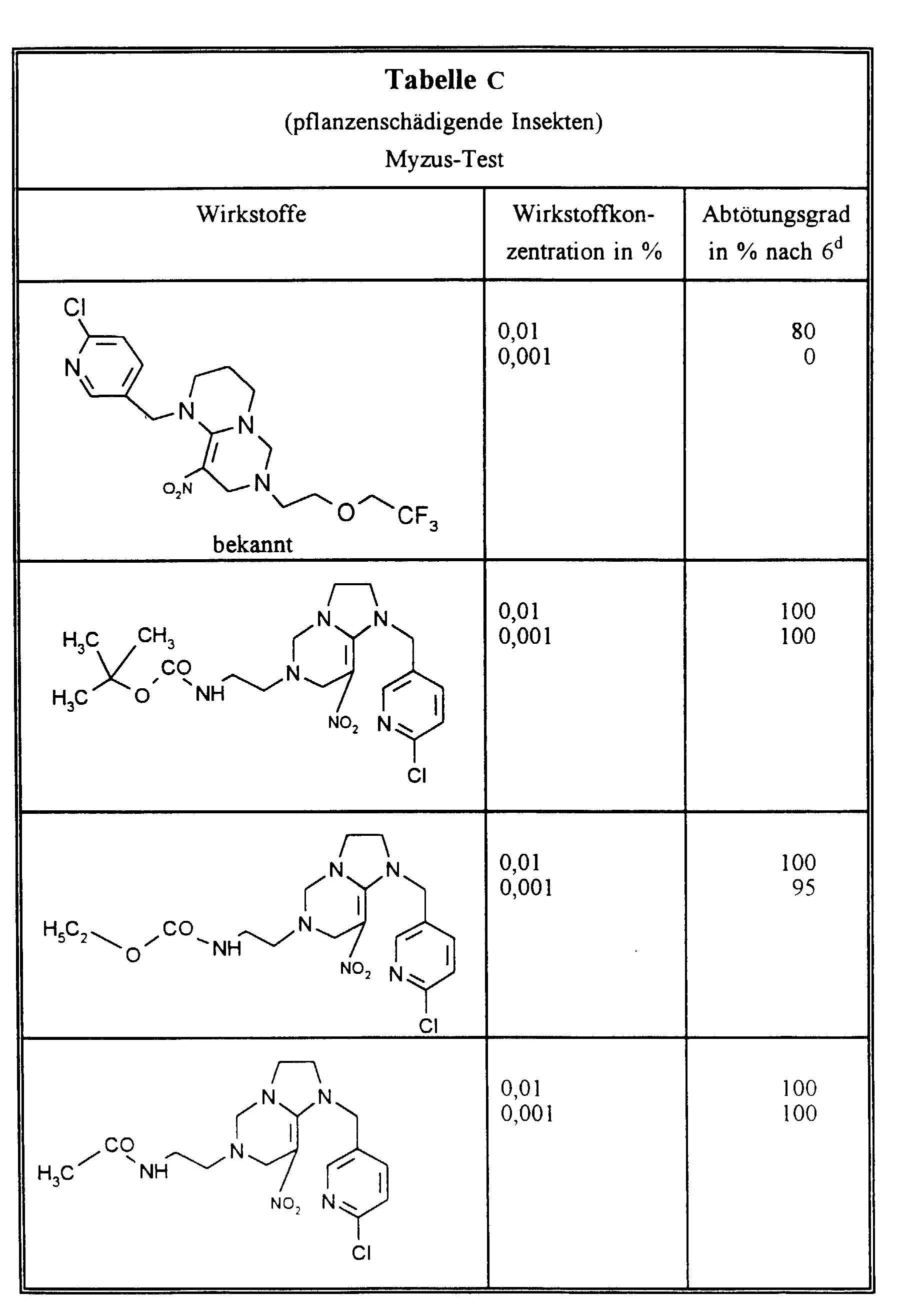

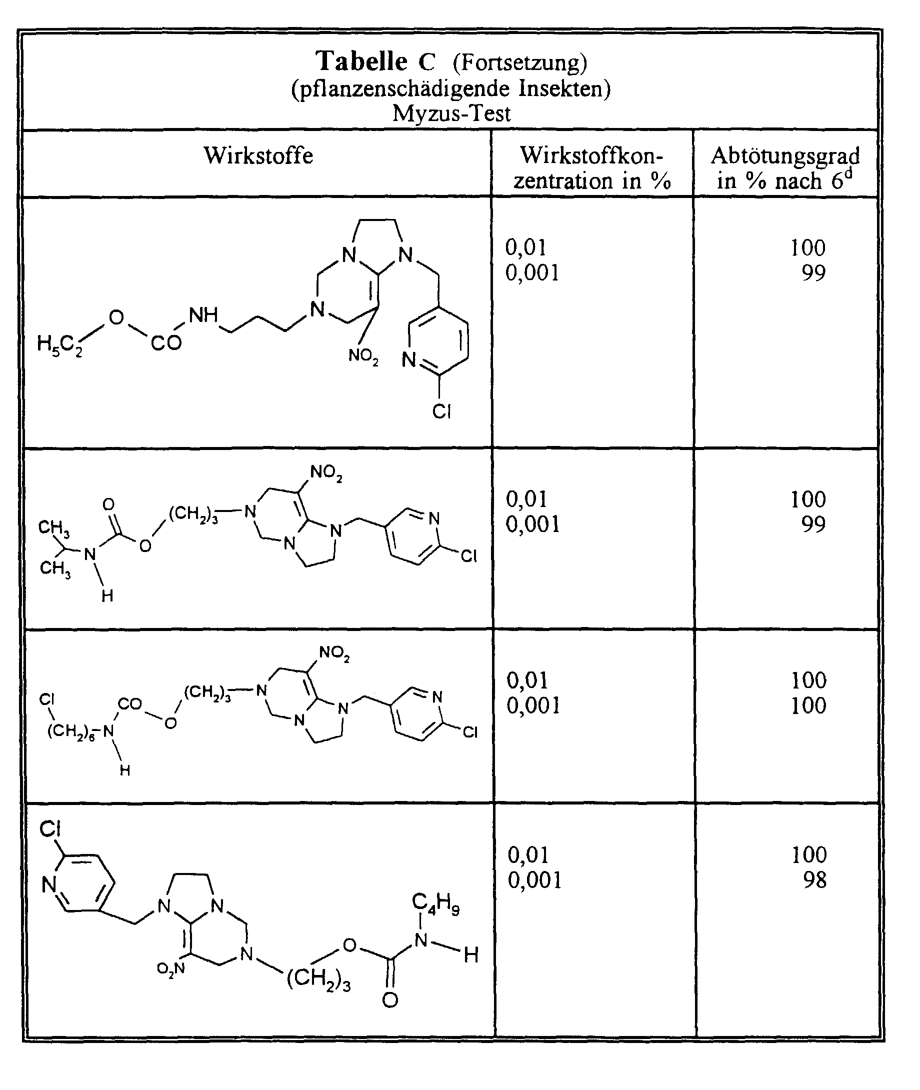

### Beispiel D

### Grenzkonzentrations-Test / Wurzelsystemische Wirkung

- Testinsekt: : Aphis fabae
- Lösungsmittel:: 4 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit vorgekeimten Dicken Bohnen. Der Wirkstoff kann so von den Pflanzenwurzeln aus dem Boden aufgenommen und in die Blätter transportiert werden.

Für den Nachweis des wurzelsystemischen Effektes werden nach 7 Tagen die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Wirkstoffe, Aufwandmengen und Resultate gehen aus der nachfolgenden Tabelle hervor:

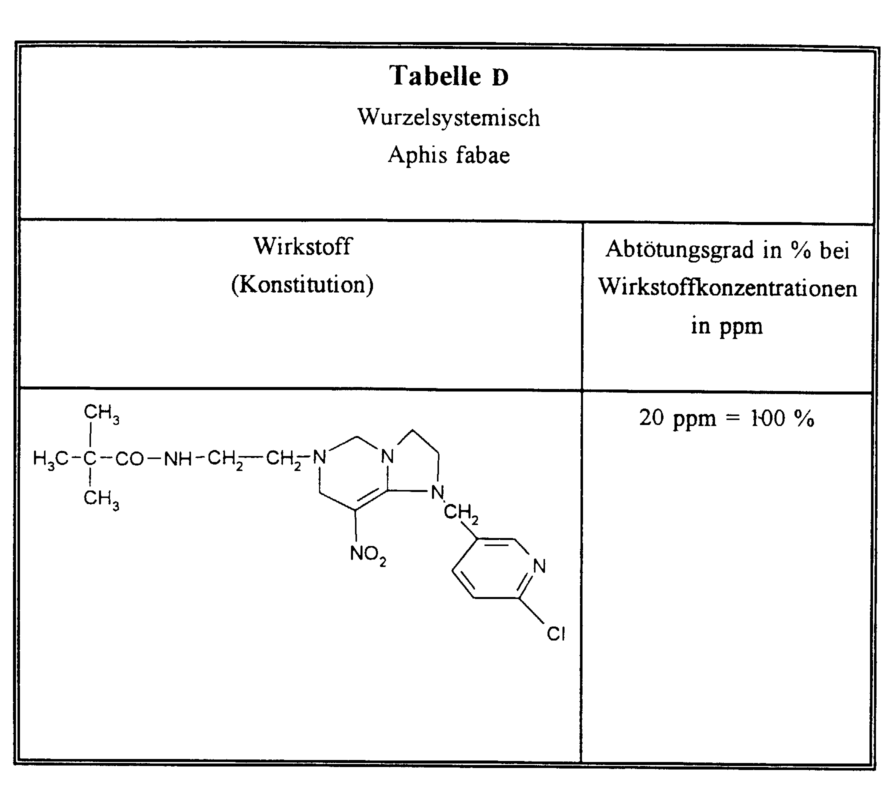

### Beispiel E

### Test mit Lucilia cuprina resistent-Larven

- Emulgator:: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der Wirkstoffzubereitung enthält. Nach 24 Stunden wird der Abtötungsgrad bestimmt.

Bei diesem Test zeigten z.B. die folgenden Verbindungen der Herstellungsbeispiele überlegene Wirkung gegenüber dem Stand der Technik und bei 100 ppm 100 % Wirkung: 3, 16, 18, 14.

### Beispiel F

### In-vitro-Test an Flöhen (alle Entwicklungsstadien)

- Testobjekt:: Alle Stadien (Eier, Larven, Puppen und Adulte) von Ctenocephalides felis.
- Testverfahren:: Blutmehl wird in einer flachen Schale über Nacht bei ca. 70°C getrocknet und anschließend mit einer Maschenweite von 0,63 mm gesiebt.
Je 1,8 g des so aufbereiteten Blutmehls werden in Plastik-Petrischalen von 9,8 cm ⌀ gegeben.
0,2 ml der Substanz werden mit der Eppendorf-Pipette auf die 1,8 g Blutmehl gegeben (Verdünnung 1:10). D.h. bei 1 ppm Anwendungskonzentration muß die wäßrige Lösung eine Konzentration von 10 ppm haben. Das Aufbringen der Lösung geschieht tropfenweise über die gesamte Oberfläche des Blutmehls verteilt.
Die so vorbereiteten Schalen über Nacht trocknen lasen. Mit einer geeigneten Vorrichtung wird die Substanz, die jetzt in getrockneten Blutmehlklumpen vorliegt, zerstoßen und durch drehende Bewegungen homogen in der Petrischale verteilt. In die so vorbereiteten Testschalen wird nun eine Spatelspitze ausgesiebter Floheier (die von künstlich infizierten Katzen stammen) gegeben.
Die Schale wird mit Parafilm verschlossen und kräftig durchgeschüttelt.
Die Inkubation geschieht bei 25°C und 85 % rel. Feuchte. Die Schalen werden in bestimmten Zeitabständen auf Entwicklungsstadien der Flöhe untersucht.
- Testkriterium:: Als Kriterium für die in-vitro-Wirkung einer Substanz gilt die Hemmung der Flohentwicklun, bzw. ein Entwicklungsstillstand vor dem Adulten-Stadium.
- Bewertung:: Wirksam: Nach der 1 1/2fachen Entwicklungszeit treten keine adulten Flöhe auf.
Unwirksam: Nach der 1 1/2fachen Entwicklungszeit treten adulte Flöhe auf.

Bei diesem Test zeigen die Verbindungen der Beispiele 3, 16, 18, 19 bei 10 ppm 100 % Wirkung.

### Fliegentest G

- Testtiere:: Musca domestica, Stamm WHO(N)
- Lösungsmittel:: 35 Gewichtsteile Ethylenglykolmonomethylether
35 Gewichtsteile Nonylphenolpolyglkolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (⌀ 9,5 cm) pipettiert, die sich in Petrischalen entsprechender Größe befinden. Nach Trocknung der Filterscheiben werden 25 Testtiere in die Petrischalen überführt und abgedeckt.

Nach 6 Stunden wird die Wirksamkeit der Wirkstoffzubereitung ermittelt. Die Wirksamkeit drückt man in % aus. Dabei bedeutet 100 %, daß alle Fliegen abgetötet wurden; 0 % bedeutet, daß keine Fliegen abgetötet wurden.

Bei diesem Beispiel zeigt die Verbindung 16 bei 1000 ppm 100 % Wirkung.

## Patentansprüche

1. Substituierte 1,2,3,4-Tetrahydro-5-nitro-pyrimidine der Formel (I) 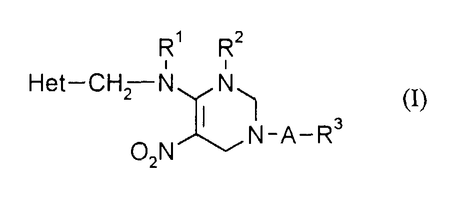 in welcher
Het für gegebenenfalls durch Halogen substituiertes Pyridyl oder für gegebenenfalls durch Halogen substituiertes Thiazolyl steht und
R¹ für Methyl oder Ethyl steht,
R² für Methyl oder Ethyl steht,
R¹ und R² gemeinsam mit den angrenzenden Atomen einen gesättigten 5- oder 6-Ring bilden der gegebenenfalls durch OH oder C₁₋₄-Alkyl substituiert ist,
A für geradkettiges oder verzweigtes Alkylen mit 2 bis 6 C-Atomen steht, das gegebenenfalls durch Phenyl, Halogen, OH, CN, Amino, Mono- oder Di-C₁₋₄-alkylamino substituiert sein kann, weiter steht A für Cyclopropylen, Methylcyclopropylen, Cyclohexylen, Cyclooctylen,
R³ für einen der Reste 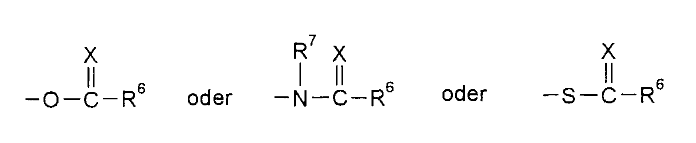 steht, wobei
R⁶ für C₁₋₈-Alkyl, C₃₋₈-Cydoalkyl, C₂₋₈-Alkenyl, Phenyl, Benzyl, Pyridyl, Imidazolyl, Pyrazolyl, Thiazolyl, Oxazolyl, Pyrrolyl, Phenylethyl, C₁₋₈-Alkoxy, C₃₋₈-Cycloalkoxy, C₃₋₈-Alkenoxy, Phenoxy, Benzyloxy, Phenylethyloxy, C₁₋₈-Alkylthio, Phenylthio, Benzylthio, Phenylethylthio, Amino, C₁₋₄-Alkylamino, Di-C₁₋₄-alkylamino, Phenylamino, N-Phenyl-N-C₁₋₄-alkylamino, Phenyl-C₁₋₄-alkyl-amino, N-Phenyl-C₁₋₄-alkyl-N-C₁₋₄-alkyl-amino steht, wobei diese Reste gegebenenfalls durch einen oder mehrere gleiche oder verschiedene Reste aus der Reihe Alkyl mit 1 bis 4 Kohlenstoffatomen, Cycloalkyl, Cycloalkylamino oder Cycloalkylalkyl mit 3 bis 7 Kohlenstoffatomen im Cycloalkylteil und 1 bis 5 Kohlenstoffatome im geradkettigen oder verzweigten Alkylteil; Phenylalkyl mit 1 bis 4 Kohlenstoffatomen im Alkylteil; Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind und als Halogenatome Fluor, Chlor oder Brom stehen, Hydroxy; Fluor, Chlor, Brom und Jod, Cyano; Nitro; Amino; Monoalkyl- und Dialkylamino oder Mono- und Dialkylaminocarbonyl mit 1 bis 4 Kohlenstoffatomen je Alkylgruppe; Carboxy; Carbalkoxy mit 2 bis 4 Kohlenstoffatomen, Sulfo (-SO₃H), Alkylsulfonyl mit 1 bis 4 Kohlenstoffatomen, Arylsulfonyl mit 6 oder 10 Arylkohlenstoffatomen substituiert sein können,
R⁷ für Wasserstoff oder C₁-C₄-Alkyl steht und
X für Sauerstoff oder Schwefel steht,
ausgenommen die Verbindung der Formel (I), in welcher Het für 6-Chlorpyrid-3-yl, A für (CH₂)₂, R³ für N(R⁷)-C(X)-R⁶, R⁷ für Wasserstoff, R⁶ für Methyl, X für Sauerstoff und R¹ und R² gemeinsam für (CH₂)₂ stehen.

2. Verbindungen der Formel (I) gemäß Anspruch 1, in welcher
Het für 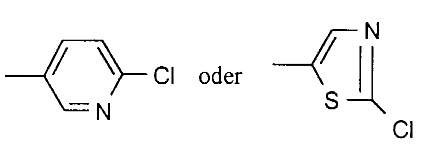 steht und
R¹ für Methyl oder Ethyl steht,
R² für Methyl oder Ethylen steht,
R¹ und R² gemeinsam mit den angrenzenden Atomen für einen gesättigten 5- oder 6-gliedrigen Ring stehen,
A für Ethylen, Propylen, Butylen, die gegebenenfalls durch Halogen insbesondere Fluor oder Chlor oder Phenyl substituiert sein können steht, ferner steht A für Cyclohexylen,
R³ für einen der Reste 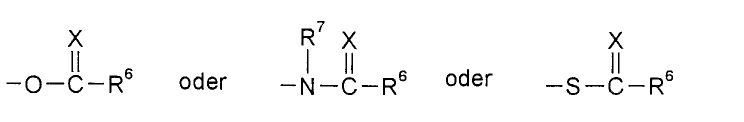 steht, wobei
R⁶ für C₁₋₆-Alkyl, Methoxy, Ethoxy, n- oder i-Propoxy, Methylamino, Dimethylamino, Ethylamino, Diethylamino, Ethylmethylamino, n-Propylamino, Di-(n-propyl)amino, Methyl(n-Propyl)amino, Ethyl(n-propyl)amino, Methylisopropylamino, Ethylisopropylamino, Isopropyl(n-propyl)amino, Cyclopentyl, Cyclohexyl, Cyclopentylmethyl, Cyclopentylethyl, Cyclopentyl-n-propyl, Cyclopentylisopropyl, Cyclohexylmethyl, Cyclohexylethyl, Cyclohexyl-n-propyl, Cylclohexylisopropyl, Phenylmethyl, Phenylethyl, Phenyl-n-propyl und/ oder Phenylisopropyl, Phenyl, Pyridyl, Imidazolyl, Thiazolyl, Oxazolyl, Phenoxy, C₁₋₄-Alkylthio oder Phenylthio steht, wobei die Reste gegebenenfalls durch C₁₋₄-Alkyl, Halogen oder CN substituiert sein können,
R⁷ für Methyl oder Wasserstoff steht und
X für Schwefel oder Sauerstoff steht,
ausgenommen die Verbindung der Formel (I), in welcher Het für 6-Chlorpyrid-3-yl, A für (CH₂)₂, R³ für N(R⁷)-C(X)-R⁶, R⁷ für Wasserstoff, R⁶ für Methyl, X für Sauerstoff und R¹ und R² gemeinsam für (CH₂)₂ stehen.

3. Verfahren zur Herstellung der substituierten 1,2,3,4-Tetrahydro-5-nitro-pyrimidine der Formel (I) gemäß Anspruch 1, dadurch gekennzeichnet, daß man
a) Nitromethylen-Derivate der Formel (II) 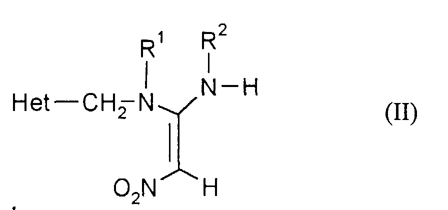 in welcher
Het, R¹ und R² die in Anspruch 1 angegebenen Bedeutungen haben,
mit Aminen der Fomel (III),
H₂N - A - R³ (III)
in welcher
A und R³ die in Anspruch 1 angegebene Bedeutung haben,
in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt, oder
b) für den Fall, daß in Formel (I) R³ für den Rest 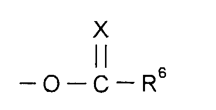 oder den Rest 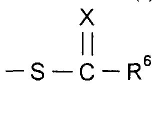 steht,
Nitromethylen-Derivate der Formeln (IV) oder (IVa) 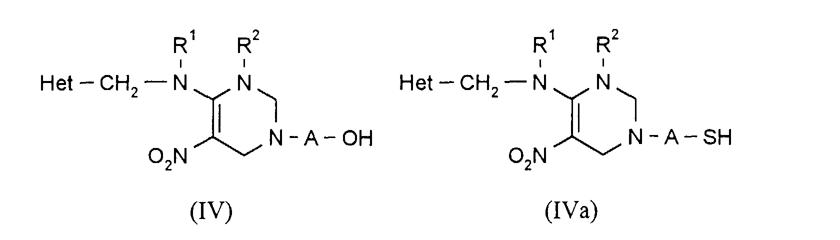 in welchen
Het, R¹, R² und A die in Anspruch 1 angegebenen Bedeutungen haben,
mit Verbindungen der Formel (V) 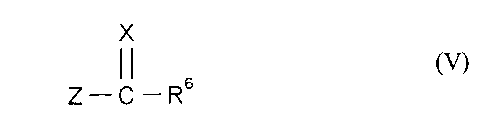 in welcher
Z für eine Abgangsgruppe oder den Rest 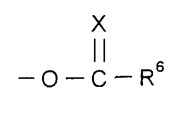 steht,
und
X und R⁶ die in Anspruch 1 angegebene Bedeutung haben,
oder mit Verbindungen der Formel (VI)
XCN - R⁹ (VI)
in welcher
X für O oder S steht,
R⁹ für einen der folgenden bei R⁶ genannten Reste Alkyl, Aryl oder Aralkyl steht,
gegebenenfalls in Gegenwart eines Säurebindemittels und gegebenenfalls in Gegenwart von Verdünnungsmitteln und/oder Katalysatoren umsetzt, oder
c) für den Fall, daß in Formel (I) R¹ und R² gemeinsam mit den angrenzenden Atomen keinen Ring schließen, man Nitromethylen-Derivate der Formel (A) 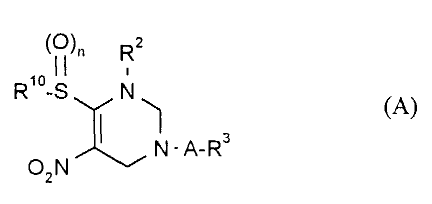 in welcher
R¹⁰ für C₁-C₄-Alkyl oder Phenyl steht,
n die Werte 0, 1 oder 2 hat,
R², R³, A die in Anspruch 1 angegebenen Bedeutungen haben,
mit Aminen der Formel (B)
Het-CH₂-NHR¹ (B)
in welcher
Het und R¹ die in Anspruch 1 angegebene Bedeutung haben,
gegebenenfalls in Gegenwart eines Säurebindemittels und gegebenenfalls von Verdünnungsmitteln und/oder Katalysatoren umsetzt.

4. Nitromethylenderivate der Formeln (IVb), (IVc) und (IVd) 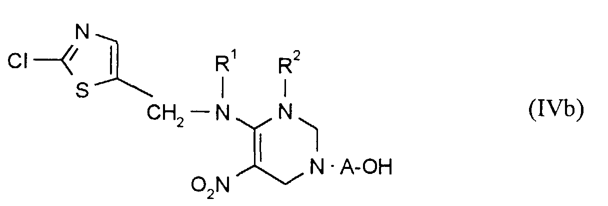 in welcher
R¹, R² und A die in Anspruch 1 angegebene Bedeutung haben, 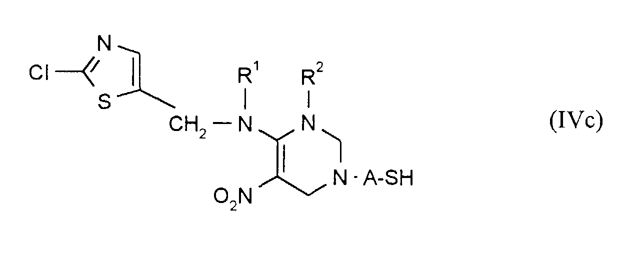 in welcher
R¹, R² und A die in Anspruch 1 angegebene Bedeutung haben, 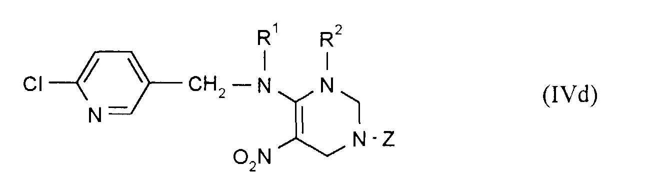 in welcher
R¹ und R² die in Anspruch 1 angegebene Bedeutung haben und
Z für 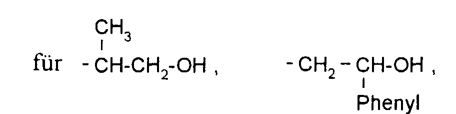 -(CH₂)₄-OH
steht.

5. Schädlingsbekämpfungsmittel und ektoparasitizide Mittel, gekennzeichnet durch einen Gehalt an mindestens einem substituierten 1,2,3,4-Tetrahydro-5-nitro-pyrimidin der Formel (I) gemäß Anspruch 1.

6. Verwendung von substituiertem 1,2,3,4-Tetrahydro-5-nitro-pyrimidin der Formel (I) gemäß Anspruch 1, zur Bekämpfung von Schädlingen und Ektoparasiten.

7. Verfahren zur Bekämpfung von Schädlingen und Ektoparasiten, dadurch gekennzeichnet, daß man substituiertes 1,2,3,4-Tetrahydro-5-nitro-pyrimidin der Formel (I) gemäß Anspruch 1 auf Schädlinge und/oder ihren Lebensraum einwirken läßt.

8. Verfahren zur Herstellung von Schädlingsbekämpfungsmitteln, dadurch gekennzeichnet, daß man substituiertes 1,2,3,4-Tetrahydro-5-nitro-pyrimidin der Formel (I) gemäß Anspruch 1 mit Streckmitteln und/oder oberflächenaktiven Mitteln vermischt.

9. Verwendung von substituiertem 1,2,3,4-Tetrahydro-5-nitro-pyrimidin der Formel (I) gemäß Anspruch 1 zur Herstellung von ektoparasitiziden Mitteln.

## Claims

1. Substituted 1,2,3,4-tetrahydro-5-nitro-pyrimidines of the formula (I) 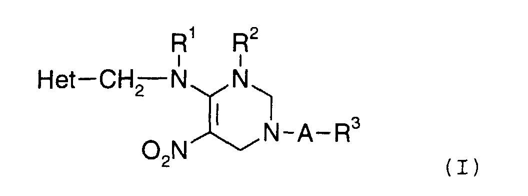 in which
Het represents pyridyl which is optionally substituted by halogen or thiazolyl which is optionally substituted by halogen and
R¹ represents methyl or ethyl,
R² represents methyl or ethyl,
R¹ and R² together with the adjacent atoms form a saturated 5- or 6-membered ring which is optionally substituted by OH or C₁₋₄-alkyl,
A represents straight-chain or branched alkylene having 2 to 6 C atoms which can optionally be substituted by phenyl, halogen, OH, CN, amino, mono- or di-C₁₋₄-alkylamino, A furthermore represents cyclopropylene, methylcyclopropylene, cyclohexylene, cyclooctylene,
R³ represents one of the radicals 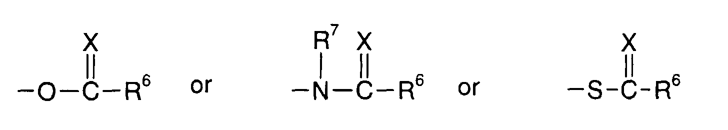
where
R⁶ represents C₁₋₈-alkyl, C₃₋₈-cycloalkyl, C₂₋₈-alkenyl, phenyl, benzyl, pyridyl, imidazolyl, pyrazolyl, thiazolyl, oxazolyl, pyrrolyl, phenylethyl, C₁₋₈-alkoxy, C₃₋₈-cycloalkoxy, C₃₋₈-alkenoxy, phenoxy, benzyloxy, phenylethyloxy, C₁₋₈-alkylthio, phenylthio, benzylthio, phenylethylthio, amino, C₁₋₄-alkylyamino, di-C₁₋₄-alkylamino, N-phenyl-N-C₁₋₄-alkylamino, phenyl-C₁₋₄-alkylamino, N-phenyl-C₁₋₄-alkyl-N-C₁₋₄-alkylamino, it being possible for these radicals optionally to be substituted by one or more identical or different radicals from the series consisting of alkyl having 1 to 4 carbon atoms, cycloalkyl, cycloalkylamino or cycloalkylalkyl having 3 to 7 carbon atoms in the cycloalkyl moiety and 1 to 5 carbon atoms in the straight-chain or branched alkyl moiety; phenylalkyl having 1 to 4 carbon atoms in the alkyl moiety; alkoxy having 1 to 4 carbon atoms, alkylthio having 1 to 4 carbon atoms, halogenoalkyl having 1 to 4 carbon atoms and 1 to 5 halogen atoms, the halogen atoms being identical or different and being fluorine, chlorine or bromine, hydroxyl; fluorine, chlorine, bromine and iodine, cyano; nitro; amino; monoalkyl- and dialkylamino or mono- and dialkylaminocarbonyl having 1 to 4 carbon atoms per alkyl group; carboxyl; carbalkoxy having 2 to 4 carbon atoms, sulpho(-SO₃H), alkylsulphonyl having 1 to 4 carbon atoms, arylsulphonyl having 6 or 10 aryl carbon atoms,
R⁷ represents hydrogen or C₁-C₄-alkyl and
X represents oxygen or sulphur,
with the exception of the compound of the formula (I), in which Het represents 6-chloro-pyrid-3-yl, A represents (CH₂)₂, R³ represents N(R⁷)-C(X)-R⁶, R⁷ represents hydrogen, R⁶ represents methyl, X represents oxygen and R¹ and R² together represent (CH₂)₂.

2. Compounds of the formula (I) according to Claim 1 in which
Het represents 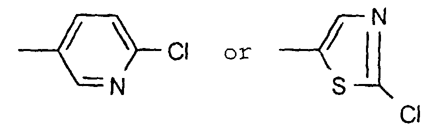 and
R¹ represents methyl or ethyl,
R² represents methyl or ethyl,
R¹ and R² together with the adjacent atoms represent a saturated 5- or 6-membered ring,
A represents ethylene, propylene, butylene, all of which can optionally be substituted by halogen, in particular fluorine or chlorine, or by phenyl, A furthermore represents cyclohexylene,
R³ represents one of the radicals 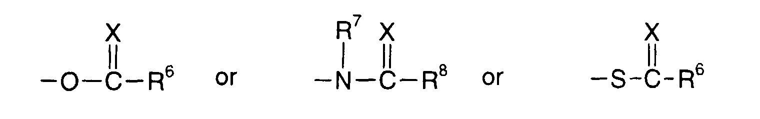 where
R⁶ represents C₁₋₆-alkyl, methoxy, ethoxy, n- or i-propoxy, methylamino, dimethylamino, ethylamino, diethylamino, ethylmethylamino, n-propylamino, di-(n-propyl)amino, methyl(n-propyl)amino, ethyl(n-propyl)amino, methylisopropylamino, ethylisopropylamino, isopropyl(n-propyl)amino, cyclopentyl, cyclohexyl, cyclopentylmethyl, cyclopentylethyl, cyclopentyl-n-propyl, cyclopentylisopropyl, cyclohexylmethyl, cyclohexylethyl, cyclohexyl-n-propyl, cyclohexylisopropyl, phenylmethyl, phenylethyl, phenyl-n-propyl and/or phenylisopropyl, phenyl, pyridyl, imidazolyl, thiazolyl, oxazolyl, phenoxy, C₁₋₄-alkylthio or phenylthio, it being possible for the radicals optionally to be substituted by C₁₋₄-alkyl, halogen or CN,
R⁷ represents methyl or hydrogen and
X represents sulphur or oxygen,
with the exception of the compound of the formula (I), in which Het represents 6-chloro-pyrid-3-yl, A represents (CH₂)₂, R³ represents N(R⁷)-C(X)-R⁶, R⁷ represents hydrogen, R⁶ represents methyl, X represents oxygen and R¹ and R² together represent (CH₂)₂.

3. Process for the preparation of the substituted 1,2,3,4-tetrahydro-5-nitro-pyrimidines of the formula (I) according to Claim 1, characterized in that
a) nitromethylene derivatives of the formula (II) 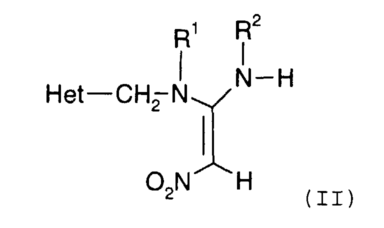 in which
Het, R¹ and R² have the meanings given in Claim 1,
are reacted with amines of the formula (III),
H₂N-A-R³ (III)
in which
A and R³ have the meanings given in Claim 1,
in the presence of at least twice the molar amount of formaldehyde, if appropriate in the presence of acidic catalysts and if appropriate in the presence of diluents, or
b) in the event that, in formula (I), R³ represents the radical 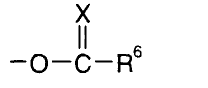 or the radical 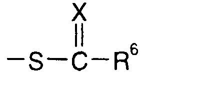 nitromethylene derivatives of the formulae (IV) or (IVa) 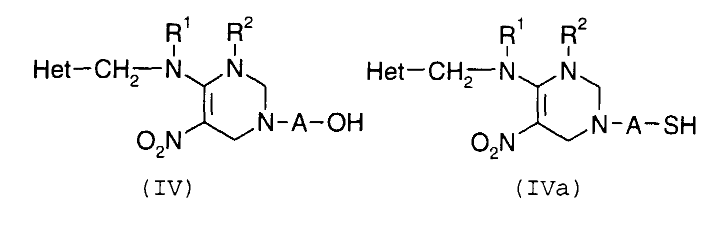 in which
Het, R¹, R² and A have the meanings given in Claim 1,
are reacted with compounds of the formula (V) 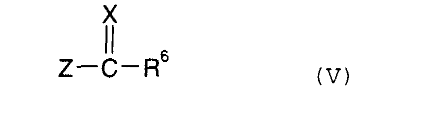 in which
Z represents a leaving group or the radical 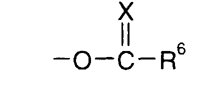 and
X and R⁶ have the meanings given in Claim 1,
or with compounds of the formula (VI)
XCN-R⁹ (VI)
in which
X represents O or S and
R⁹ represents one of the following radicals alkyl, aryl or aralkyl which have been mentioned under R⁶,
if appropriate in the presence of an acid-binding agent and if appropriate in the presence of diluents and/or catalysts, or
c) in the event that, in formula (I), R¹ and R² together with the adjacent atoms do not cyclize, nitromethylene derivatives of the formula (A) 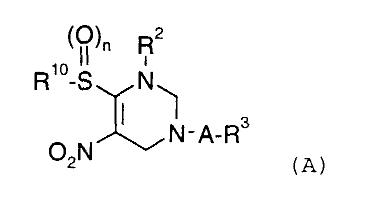 in which
R¹⁰ represents C₁-C₄-alkyl or phenyl,
n assumes values of 0, 2 or 2 and
R², R³ and A have the meanings given in Claim 1,
are reacted with amines of the formula (B)
Het-CH₂-NHR¹ (B)
in which
Het and R¹ have the meanings given in Claim 1,
if appropriate in the presence of an acid-binding agent and if appropriate of diluents and/or catalysts.

4. Nitromethylene derivatives of the formula (IVb), (IVc) and IVd) 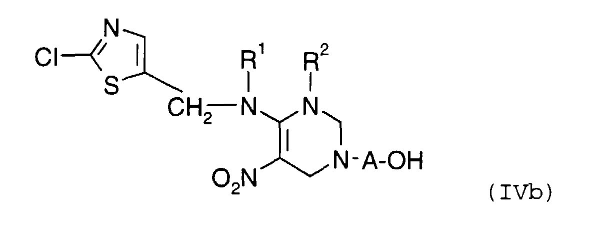 in which
R¹, R² and A have the meanings given in Claim 1, 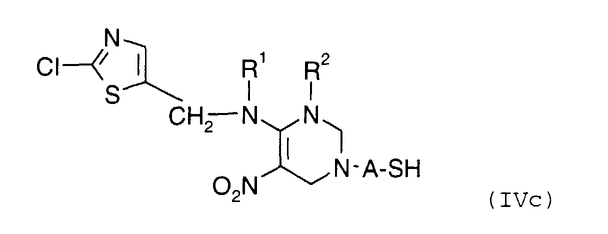 in which
R¹, R² and A have the meanings given in Claim 1, 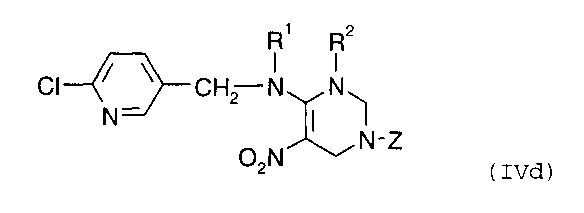 in which
R¹ and R² have the meanings given in Claim 1 and
z represents

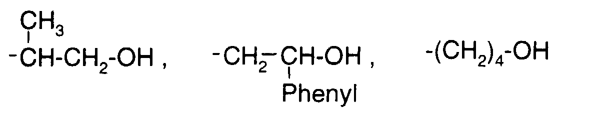

5. Pesticides and ectoparasiticidal compositions, characterized in that they contain at least one substituted 1,2,3,4-tetrahydro-5-nitro-pyridine of the formula (I) according to Claim 1.

6. Use of substituted 1,2,3,4-tetrahydro-5-nitro-pyrimidine of the formula (I) according to Claim 1 for combating pests and ectoparasites.

7. Method of combating pests and ectoparasites, characterized in that substituted 1,2,3,4-tetrahydro-5-nitro-pyrimidine of the formula (I) according to Claim 1, is allowed to act on pests and/or their environment.

8. Process for the preparation of pesticides, characterized in that substituted 1,2,3,4-tetrahydro-5-nitro-pyrimidine of the formula (I) according to Claim 1 is mixed with extenders and/or surface-active agents.

9. Use of substituted 1,2,3,4-tetrahydro-5-nitro-pyrimidine of the formula (I) according to Claim 1 for the preparation of ectoparasiticidal compositions.

## Revendications

1. 1,2,3,4-tétrahydro-5-nitropyrimidines substituées de formule (I) 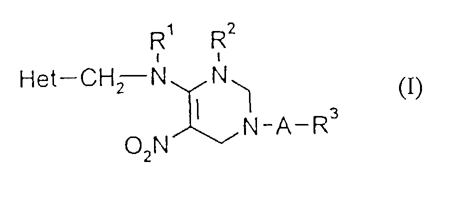 dans laquelle
Het représente un groupe pyridyle éventuellement substitué par un halogène ou un groupe thiazolyle éventuellement substitué par un halogène et
R¹ représente un groupe méthyle ou éthyle,
R² représente un groupe méthyle ou éthyle,
R¹ et R² forment conjointement avec les atomes adjacents un noyau pentagonal ou hexagonal saturé qui est substitué, le cas échéant par un radical OH ou alkyle en C₁ à C₄,
A est un groupe alkylène linéaire ou ramifié ayant 2 à 6 atomes de carbone, qui peut être substitué, le cas échéant par un radical phényle, halogéno, OH, CN, amino, mono- ou di-(alkyle en C₁ à C₄)amino, en outre A représente un groupe cyclopropylène, méthylcyclopropylène, cyclo-hexylène, cyclo-octylène,
R³ représente l'un des restes 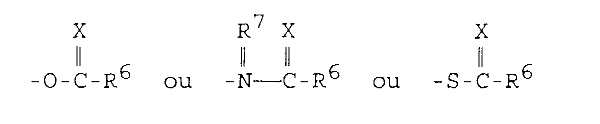 dans lesquels
R⁶ est un groupe alkyle en C₁ à C₈, cycloalkyle en C₃ à C₈, alcényle en C₂ à C₈, phényle, benzyle, pyridyle, imidazolyle, pyrazolyle, thiazolyle, oxazolyle, pyrrolyle, phényléthyle, alkoxy en C₁ à C₈, cycloalkoxy en C₃ à C₈, alcénoxy en C₃ à C₈, phénoxy, benzyloxy, phényléthyloxy, alkylthio en C₁ à C₈, phénylthio, benzylthio, phényléthylthio, amino, alkylamino en C₁ à C₄, di(alkyle en Cₗ à C₄)amino, phénylamino, N-phényl-N-(alkyle en C₁ à C₄)amino, phényl-(alkyle en C₁ à C₄)amino, N-phényl-(alkyle en C₁ à C₄)-N-(alkyle en C₁ à C₄)amino, ces restes pouvant être substitués, le cas échéant par un ou plusieurs restes identiques ou différents de la série alkyle ayant 1 à 4 atomes de carbone, cycloalkyle, cycloalkylamino ou cycloalkylalkyle ayant 3 à 7 atomes de carbone dans la partie cycloalkyle et 1 à 5 atomes de carbone dans la partie alkyle linéaire ou ramifiée ; phénylalkyle ayant 1 à 4 atomes de carbone dans la partie alkyle ; alkoxy ayant 1 à 4 atomes de carbone, alkylthio ayant 1 à 4 atomes de carbone, halogénalkyle ayant 1 à 4 atomes de carbone et 1 à 5 atomes d'halogènes, les atomes d'halogènes étant identiques ou différents et étant représentés par le fluor, le chlore ou le brome, hydroxy ; fluor, chlore, brome et iode, cyano ; nitro ; amino ; monoalkyl- et dialkylamino ou mono- et dialkylaminocarbonyle ayant 1 à 4 atomes de carbone dans chaque groupe alkyle, carboxy ; carbalkoxy ayant 2 à 4 atomes de carbone, sulfo-(-SO₃H), alkylsulfonyle ayant 1 à 4 atomes de carbone, arylsulfonyle ayant 6 ou 10 atomes de carbone dans le groupe aryle,
R⁷ est l'hydrogène ou un groupe alkyle en C₁ à C₄ et
X est l'oxygène ou le soufre,
à l'exception du composé de formule (I) dans laquelle Het est le groupe 6-chloropyrid-3-yle, A est le groupe (CH₂)₂, R³ est un groupe N(R⁷)-C(X)-R⁶, R⁷ est l'hydrogène, R⁶ est le groupe méthyle, X est l'oxygène et R¹ et R² forment conjointement un groupe (CH₂)₂.

2. Composés de formule (I) suivant la revendication 1, dans laquelle
Het représente 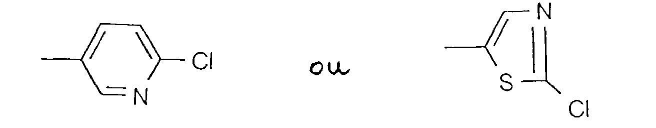 et
R¹ est un groupe méthyle ou éthyle,
R² est un groupe méthyle ou éthylène,
R¹ et R² forment conjointement avec les atomes adjacents un noyau saturé pentagonal ou hexagonal,
A représente des groupes éthylène, propylène, butylène qui peuvent être substitués, le cas échéant par un halogène, notamment le fluor ou le chlore, ou par un radical phényle, en outre A représente un groupe cyclohexylène,
R³ est l'un des restes 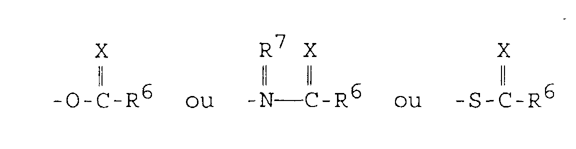 où
R⁶ est un groupe alkyle en C₁ à C₆, méthoxy, éthoxy, n- ou isopropoxy, méthylamino, diméthylamino, éthylamino, diéthylamino, éthylméthylamino, n-propylamino, di-(n-propyl)amino, méthyl-(n-propyl)amino, éthyl-(n-propyl)amino, méthylisopropylamino, éthylisopropylamino, isopropyl-(n-propyl)amino, cyclopentyle, cyclohexyle, cyclopentylméthyle, cyclopentyléthyle, cyclopentyl-n-propyle, cyclopentylisopropyle, cyclohexylméthyle, cyclohexyléthyle, cyclohexyl-n-propyle, cyclohexylisopropyle, phénylméthyle, phényléthyle, phényl-n-propyle et/ou phénylisopropyle, phényle, pyridyle, imidazolyle, thiazolyle, oxazolyle, phénoxy, alkylthio en C₁ à C₄ ou phénylthio, les restes pouvant être substitués, le cas échéant par un radical alkyle en C₁ à C₄, halogéno ou CN,
R⁷ est un groupe méthyle ou l'hydrogène et
X est le soufre ou l'oxygène,
excepté le composé de formule (I) dans laquelle Het est un groupe 6-chloropyrid-3-yle, A est un groupe (CH₂)₂, R³ est un groupe N(R⁷)-C(X)-R⁶, R⁷ est l'hydrogène, R⁶ est le groupe méthyle, X est l'oxygène et R¹ et R² forment conjointement un groupe (CH₂)₂.

3. Procédé de production des 1,2,3,4-tétrahydro-5-nitropyrimidines substituées de formule (I) suivant la revendication 1, caractérisé en ce que
a) l'on fait réagir des dérivés nitrométhyléniques de formule (II) 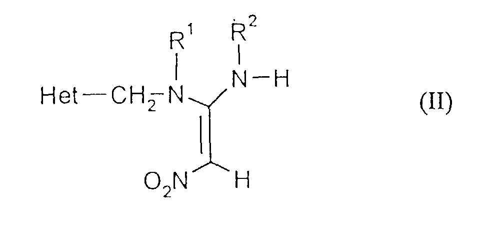 dans laquelle
Het, R¹ et R² ont les définitions indiquées dans la revendication 1,
avec des amines de formule (III)
H₂N - A - R³ (III)
dans laquelle
A et R³ ont la définition indiquée dans la revendication 1,
en présence d'au moins le double de la quantité molaire de formaldéhyde, le cas échéant en présence de catalyseurs acides et en la présence éventuelle de diluants, ou bien
b) au cas où dans la formule (I) R³ représente le reste 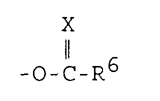 ou le reste 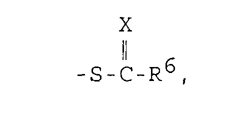 on fait réagir des dérivés nitrométhyléniques de formules (IV) ou (IVa) 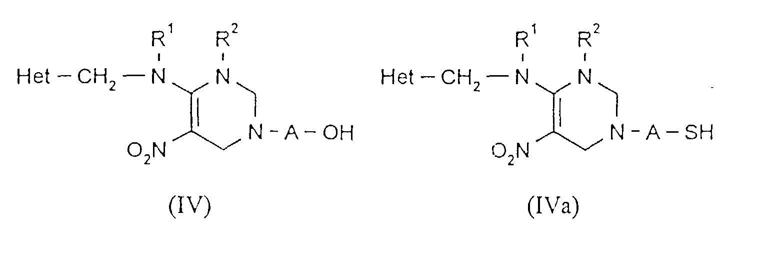 dans lesquelles
Het, R¹, R² et A ont les définitions indiquées dans la revendication 1,
avec des composés de formule (V) 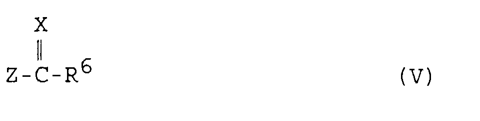 dans laquelle
Z représente un groupe partant ou le reste 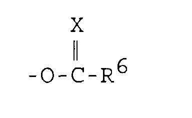 et
X et R⁶ ont la définition indiquée dans le revendication 1,
ou avec des composés de formule (VI)
XCN - R⁹ (VI)
dans laquelle
X représente O ou S,
R⁹ représente l'un des restes alkyle, aryle ou aralkyle mentionnés pour R⁶,
le cas échéant en présence d'un accepteur d'acide et en la présente éventuelle de diluants et/ou de catalyseurs, ou bien
c) au cas où dans la formule (I), R¹ et R² ne forment pas de noyau conjointement avec les atomes adjacents, on fait réagir des dérivés nitrométhyléniques de formule (A) 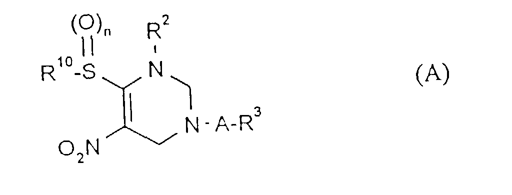 dans laquelle
R⁰ est un groupe alkyle en C₁ à C₄ ou phényle,
n a les valeurs 0, 1 ou 2,
R², R³, A ont les définitions indiquées dans la revendication 1,
avec des amines de formule (B)
Het-CH₂-NHR¹ (B)
dans laquelle
Het et R¹ ont la définition indiquée dans la revendication 1,
le cas échéant en présence d'un accepteur d'acide et en la présence éventuelle de diluants et/ou de catalyseurs.

4. Dérivés nitrométhyléniques de formules (IVb), (IVc) et (IVd) 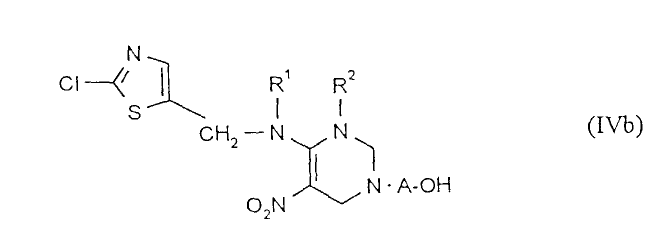 dans lesquelles
R¹, R² et A ont la définition indiquée dans la revendication 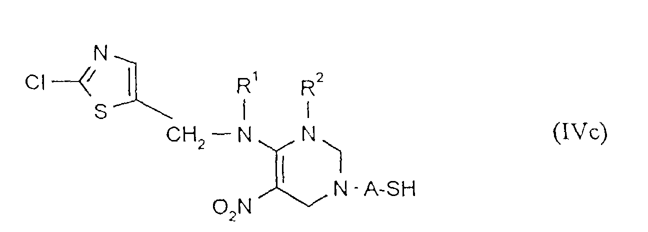
dans laquelle
R¹, R² et A ont la définition indiquée dans la revendication 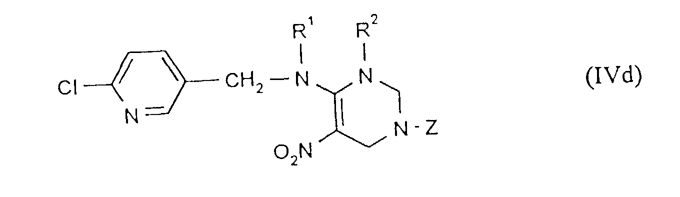
dans laquelle
R¹ et R² ont la définition indiquée dans la revendication 1 et
Z représente 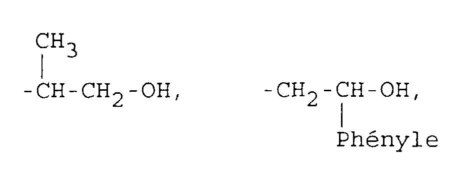 -(CH₂)₄-OH

5. Compositions pesticides et compositions ectoparasiticides, caractérisées par une teneur en au moins une 1,2,3,4-tétrahydro-5-nitropyrimidine substituée de formule (I) suivant la revendication 1.

6. Utilisation de 1,2,3,4-tétrahydro-5-nitro-pyrimidine substituée de formule (I) suivant la revendication 1 pour combattre des parasites et des ectoparasites.

7. Procédé pour combattre des parasites et des ectoparasites, caractérisé en ce qu'on fait agir une 1,2,3,4-tétrahydro-5-nitropyrimidine substituée de formule (I) suivant la revendication 1 sur des parasites et/ou sur leur milieu.

8. Procédé de préparation de compositions pesticides, caractérisé en ce qu'on mélange une 1,2,3,4-tétrahydro-5-nitropyrimidine substituée de formule (I) suivant la revendication 1 avec des diluants et/ou des agents tensio-actifs.

9. Utilisation de 1,2,3,4-tétrahydro-5-nitro-pyrimidine substituée de formule (I) suivant la revendication 1 pour la préparation de compositions ectoparasiticides.
